# EUROPÄISCHE PATENTSCHRIFT

(11) **EP 2 389 400 B1**
(45) Veröffentlichungstag und Bekanntmachung des Hinweises auf die Patenterteilung: **05.12.2012**
(21) Anmeldenummer: 10700496.2
(22) Anmeldetag: 13.01.2010
(51) Int. Cl.: C08G 18/08, C08G 18/10, C08G 18/12, C08G 18/28, C08G 18/40, C08G 18/42, C08G 18/44, C08G 18/48, C08G 18/72, A61L 15/22, C08J 9/30, A61L 15/26, A61L 15/42

(54) **VERWENDUNG ZWEIKOMPONENTIGER POLYURETHAN-DISPERSIONSSCHÄUME ALS WUNDAUFLAGEN**
USE OF DISPERSED TWO-COMPONENT POLYURETHANE FOAMS AS wound dressing
UTILISATION DE MOUSSES DE DISPERSIONS POLYURÉTHANE À DEUX COMPOSANTS COMME PANSEMENT POUR PLAIE

(30) Priorität: 24.01.2009 EP 09000992
(43) Veröffentlichungstag der Anmeldung: 30.11.2011
(73) Patentinhaber: Bayer MaterialScience AG, 51368 Leverkusen (DE)
(72) Erfinder: SCHÖNBERGER, Jan, 42781 Haan (DE); DÖRR, Sebastian, 40593 Düsseldorf (DE)
(86) Internationale Anmeldenummer: PCT/EP2010/000121
(87) Internationale Veröffentlichungsnummer: WO 2010/083953

(56) Entgegenhaltungen:
- WO-A-2008/031520
- DE-A1-102006 016 639
- DE-A1-102006 020 745

## Beschreibung

Die Erfindung betrifft die Verwendung von Polyurethan-Schäumen, die nach einem Verfahren erhältlich sind, bei dem eine Zusammensetzung, enthaltend eine anionische und eine kationische Polyurethan-Dispersion aufgeschäumt und getrocknet werden, als Wundauflagen.

Die Verwendung von Wundauflagen aus Schäumen zur Behandlung von nässenden Wunden ist Stand der Technik. Aufgrund ihres hohen Absorptionsvermögens und ihrer guten mechanischen Eigenschaften werden in Regel Polyurethan-Schäume eingesetzt, welche durch Umsetzung von Mischungen aus Diisocyanate und Polyolen bzw. NCO-funktionellen Polyurethan-Prepolymeren mit Wasser in Gegenwart bestimmter Katalysatoren sowie (Schaum-) Additiven hergestellt werden. In der Regel werden hierbei aromatische Diisocyanate eingesetzt, da sich diese am besten Verschäumen lassen. Zahlreiche Ausführungsformen dieser Verfahren sind bekannt, beispielsweise beschrieben in US 3,978,266, US 3,975,567 und EP-A 0 059 048. Die vorgenannten Verfahren weisen jedoch den Nachteil auf, dass reaktive Mischungen eingesetzt werden müssen, enthaltend Diisocyanate oder entsprechende NCO-funktionelle Prepolymere, deren Handhabung technisch aufwendig ist, da z.B. entsprechende Schutzmaßnahmen erforderlich sind.

Eine Alternative zum vorstehend beschriebenen Verfahren, in welchem Diisocyanate bzw. NCO-funktionelle Polyurethan-Prepolymere eingesetzt werden, ist ein Verfahren basierend auf Polyurethan-Dispersionen (die im Wesentlichen frei von Isocyanat-Gruppen sind), in welche man in Gegenwart geeigneter (Schaum-) Additive durch kräftiges Rühren Luft einträgt. Nach dem Trocknen und Aushärten werden so genannte mechanische Polyurethan-Schäume erhalten. Solche Schäume sind im Zusammenhang mit Wundauflagen beschrieben in EP-A 0 235 949 und EP-A 0 246 723, wobei dem Schaum entweder ein selbsthaftendes Polymer zugesetzt oder der Schaum auf einen Film eines selbsthaftenden Polymers aufgebracht wird. Die in EP-A 0 235 949 und EP-A 0 246 723 aufgeführten Beispiele beschreiben darüber hinaus zwingend die Verwendung von Polyaziridinen als Vernetzer, was jedoch nach heutigem Stand des Wissens hinsichtlich deren Toxizität nicht mehr akzeptabel ist. Zur Versetzung müssen überdies hohe Einbrenntemperaturen angewandt werden, angegeben sind 100°C bis 170°C. In US 4,655,210 ist die Verwendung der vorgenannten mechanischen Schäume für Wundauflagen beschrieben, die einen speziellen Aufbau aus Träger, Schaum und Hautkontakt-Schicht aufweisen. Die nach den in EP-A 0 235 949 und EP-A 0 246 723 beschriebenen Verfahren hergestellten Schäume weisen darüber hinaus den großen Nachteil auf, dass die erhaltenen Schäume nur in geringem Maße offenzellig sind, wodurch die Absorption von physiologischer Salzlösung sowie auch die Wasserdampfdurchlässigkeit gering sind.

Zur Versorgung von Wunden mit komplexer Topologie oder zur Bedeckung besonders tiefer Wunden ist die Verwendung gebrauchsfertiger, industriell hergestellter flächiger Wundauflagen sehr schwierig, da eine optimale Bedeckung der Wundoberfläche in der Regel nicht gelingt, wodurch der Heilungsprozess verzögert wird. Um eine bessere Bedeckung tiefer Wunden zu erreichen, wurde vorgeschlagen, Granulate aus mikroporösen Polyurethanen anstelle kompakter Wundauflagen einzusetzen (EP-A-0 171 268). Jedoch wird auch hierdurch keine optimale Bedeckung der Wunde erreicht.

Der Auftrag einer (fließfähigen) Zusammensetzung, welche sich der Wundform optimal anpasst, würde die Nachteile flächiger Wundauflagen beseitigen. Die beiden vorstehend beschriebenen Verfahren, in welchen zur Herstellung der Polyurethan-Schäume entweder Diisocyanate bzw. NCO-funktionelle Polyurethan-Prepolmere oder Polyurethan-Dispersionen in Kombination mit Polyaziridinen eingesetzt werden, sind hierzu jedoch unbrauchbar: reaktive Zusammensetzungen, die freie Isocyanat-Gruppen enthalten, können aus toxikologischen Gründen nicht direkt auf die Haut aufgetragen werden, auch wenn dies verschiedentlich vorgeschlagen wurde (siehe beispielsweise WO 02/26848). Aber auch die Verwendung von Polyurethan-Dispersionen mit Polyaziridinen als Vernetzer ist aufgrund der nach heutigem Stand des Wissens toxikologisch bedenklichen Eigenschaften des Vernetzers ausgeschlossen.

Ein Verfahren zur raschen, nicht thermisch initiierten Verfestigung geschäumter Polyurethan-Dispersionen ist bislang nicht bekannt, auch wenn die Herstellung von Polyurethan-Filmen, d.h. nicht-porösen Materialien, durch z.B. Koagulation mit anorganischen Saken ein weithin technisch gebräuchliches Verfahren ist.

Die DE 10 2006 020745 beschreibt mikroporösen Beschichtungen basierend auf Polyurethan-Schäumen, die durch Aufschäumen und Trocknen einer Zusammensetzung, die erhältlich ist durch Vermischen von einer anionisch hydrophilierten und einer kationisch hydrophilierten Polyurethan-Dispersion erhältlich sind. Als Anwendungsgebiete werden die Bereiche Oberbekleidung, Kunstlederartikel, Schuhe, Möbelbezugstoffe, Automobil-Innenausstattungsartikel und Sportgeräte offenbart.

Aufgabe der vorliegenden Erfindung war daher die Bereitstellung von rasch verfestigenden Polyurethan-Schäumen, insbesondere für die Wundbehandlung, wobei die Herstellung unter Verwendung einer Zusammensetzung erfolgt, die frei von Isocyanat-Gruppen ist. Die Herstellung des Polyurethan-Schaums soll grundsätzlich auch bei Umgebungsbedingungen durchgeführt werden können, wobei die gebildeten Polyurethan-Schäume ohne merkliche Entwicklung von Reaktionswärme und innerhalb kürzester Zeit ausreichende mechanische Eigenschaften aufweisen sollen-Die Zusammensetzung soll darüber hinaus zum direkten Auftrag auf die Haut geeignet sein, z.B. durch Sprühen oder Gießen, damit die Wunde optimal mit dem Polyurethan-Schaum bedeckt wird. Eine optimale Wundabdeckung erfordert zudem keinen oder lediglich einen geringen Volumenschrumpf der Polyurethan-Schäume während und nach der Applikation.

Es wurde nun überraschend gefunden, dass es durch die Kombination von speziellen anionisch hydrophilierten PUR-Dispersionen (I) und speziellen kationisch hydrophilierten PUR-Dispersionen (II) gelingt, sich innerhalb weniger Sekunden und ohne messbare Wärmetönung verfestigende Schäume zu erhalten.

Wundauflagen aus Polyurethan-Schäumen im Sinne der Erfindung sind poröse Materialien, bevorzugt mit zumindest teilweise vorhandener Offenzelligkeit, welche im Wesentlichen aus Polyurethanen bestehen und Wunden im Sinne einer sterilen Abdeckung vor Keimen bzw. Umgebungseintlüssen schützen, durch geeignete Feuchtedurchlässigkeit für ein geeignetes Wundklima sorgen und eine ausreichende mechanische Festigkeit aufweisen.

Gegenstand der vorliegenden Erfindung ist daher die Verwendung von Polyurethan-Schäumen erhältlich nach einem Verfahren, bei dem eine Zusammensetzung erhältlich durch Vermischen von wenigstens einer wässrigen, anionisch hydrophilierten Polyurethan-Dispersion (T) und wenigstens einer kationisch hydrophitierten PUR-Dispersion (II) bereitgestellt und während oder nach der vollständigen Vermischung der Einzelkomponenten aufgeschäumt und ausgehärtet wird, als Wundauflage.

Aushärten in Bezug auf die Schaumherstellung meint eine Verfestigung der enthaltenen Polymere u.a. durch Trocknung, also Entzug von Wasser und/oder Koagulation. Ein solcher verfestigter Schaum kann damit auch ein noch (wasser-)feuchtes, jedoch bereits in sich festes geschäumtes Material sein.

Bevorzugte sind Zusammensetzungen, bei deren Herstellung anionisch hydrophilierte Polyurethan-Dispersionen (1) mit einem Gehalt an -COO- oder -SO₃ oder -PO₂²⁻ Gruppen von 2 bis 500 Milliäquivalenlen pro 100 g Festharz und kationisch hydrophilierte Polyurethan-Dispersionen (II) mit einem Gehalt an quaternären Ammoniumgruppen von 2 bis 500 Milliäquivalenten pro 100 g Festharz verwendet wurden.

Neben den Dispersionen (f) und (TI) können die erfindungswesentlichen Zusammensetzungen Hilfs- und Zusatzstoffe (III) mitumfassen.

Die in den erfindungswesentlichen Zusammensetzungen enthaltenen wässrigen, anionisch hydrophilierten Polyurethan-Dispersionen (I) sind erhältlich, indem
A) isocyanatfunktionelle Prepolymere aus
   A1) organischen Polyisocyanaten
   A2) polymeren Polyolen mit zahlenmittleren Molekulargewichten von 400 bis 8000 g/mol, vorzugsweise 400 bis 6000 g/mol und besonders bevorzugt von 600 bis 3000 g/mol, und OH-Funktionalitäten von 1,5 bis 6, bevorzugt 1,8 bis 3, besonders bevorzugt von 1,9 bis 2,1, und
   A3) gegebenenfalls hydroxyfunktionellen Verbindungen mit Molekulargewichten von 62 bis 399 g/mol und
   A4) gegebenenfalls isocyanatreaktiven, anionischen oder potentiell anionischen und/oder gegebenenfalls nichtionischen Hydrophilierungsmitteln,
   hergestellt werden,
B) deren freie NCO-Gruppen dann ganz oder teilweise
   B1) gegebenenfalls mit aminofunktionellen Verbindungen mit Molekulargewichten von 32 bis 400 g/mol und
   B2) gegebenenfalls mit isocyanatreaktiven, bevorzugt aminofunktionellen, anionischen oder potentiell anionischen Hydrophilierungsmitteln
unter Kettenverlängerung umgesetzt werden und die Prepolymere vor während oder nach Schritt B) in Wasser dispergiert werden, wobei gegebenenfalls enthaltene potentiell anionische Gruppen durch teilweise oder vollständige Umsetzung mit einem Neutralisationsmittel in die anionische Form überführt werden.

Um eine anionische Hydrophilierung zu erreichen, müssen in A4) und/oder B2) Hydrophilierungsmittel eingesetzt werden, die wenigstens eine gegenüber NCO-Gruppen reaktive Gruppe wie Amino-, Hydroxy- oder Thiolgruppen aufweisen und darüber hinaus -COO- oder -SO₃⁻ oder -PO₃²⁻ als anionische bzw. deren ganz oder teilweise protonierte Säureformen als potentiell anionische Gruppen aufweisen.

Bevorzugte wässrige, anionische Polyurethan-Dispersionen (I) haben einen niedrigen Grad an hydrophilen anionischen Gruppen, bevorzugt von 2 bis 200 Milliäquivalenten, besonders bevorzugt von weniger als 10 bis 100 Milliäquivalenten pro 100 g Festharz.

Um eine gute Sedimentationsstabilität zu erreichen, liegt die zahlenmittlere Teilchengröße der Polyurethan-Dispersionen (I) bevorzugt bei weniger als 750 nm, besonders bevorzugt bei weniger als 500 nm, bestimmt mittels Laserkorrelations-Spektroskopie.

Das Verhältnis von NCO-Gruppen der Verbindungen aus Komponente A1) zu NCO-reaktiven Gruppen wie Amino-, Hydroxy- oder Thiolgruppen der Verbindungen der Komponenten A2) bis A4) beträgt bei der Herstellung des NCO-funktionellen Prepolymers 1,05 bis 3,5; bevorzugt 1,2 bis 3,0 und besonders bevorzugt 1,3 bis 2,5.

Die aminofunktionellen Verbindungen in Stufe B) werden in solch einer Menge eingesetzt, dass das Äquivalentverhältnis von isocyanatreaktiven Aminogruppen dieser Verbindungen zu den freien Isocyanatgruppen des Prepolymers 40 bis 150 %, bevorzugt 50 bis 125 %, besonders bevorzugt 60 bis 100 % beträgt.

Geeignete Polyisocyanate der Komponente A1) sind die dem Fachmann an sich bekannten aromatischen, araliphatischen, aliphatischen oder cycloaliphatischen Polyisocyanate einer NCO-Funktionalität von ≥2.

Beispiele solcher geeigneten Polyisocyanate sind 1,4-Butylendiisocyanat, 1,6-Hexamethylendiisocyanat (HDI), Isophorondiisocyanat (IPDI), 2,2,4- und/oder 2,4,4- Trimethylhexamethylendiisocyanat, die isomeren Bis(4,4`-isocyanatocyclohexyl)methane oder deren Mischungen beliebigen Isomerengehalts, 1,4-Cyclohexylendüsocyanat, 1,4-Phenylendiisocyanat, 2,4- und/oder 2,6-Toluylendüsocyanat, 1,5-Naphthylendiisocyanat, 2,2`-und/oder 2,4`- und/oder 4,4`-diphenylmethandiisocyanat, 1,3- und/oder 1,4-Bis-(2-isocyanato-prop-2-yl)-benzol (TMXDI), 1,3-Bis-(isocyanatomethyl)benzol (XDn, sowie Alkyl-2,6-diisocyanatohexanoate (Lysindiisocyanate) mit C1-C8-Alkylgruppen.

Neben den vorstehend genannten Polyisocyanaten können anteilig auch modifizierte Diisocyanate mit Uretdion-, Isocyanurat-, Urethan-, Allophanat-, Biuret-, Iminooxadiazindion- und/oder Oxadiazintrionstruktur sowie nicht-modifiziertes Polyisocyanat mit mehr als 2 NCO-Gruppen pro Molekül wie z.B. 4-Isocyanatomethyl-1,8-octandiisocyanat (Nonantriisocyanat) oder Triphenylmethan-4,4',4"-triisocyanat mit eingesetzt werden.

Bevorzugt handelt es sich um Polyisocyanate oder Polyisocyanatgemische der vorstehend genannten Art mit ausschließlich aliphatisch und/oder cycloaliphatisch gebundenen Isocyanatgruppen und einer mittleren NCO-Funktionalität der Mischung von 2 bis 4, bevorzugt 2 bis 2,6 und besonders bevorzugt 2 bis 2,4.

Besonders bevorzugt werden in A1) 1,6-Hexamethylendiisocyanat, Isophorondiisocyanat, die isomeren Bis(4,4'-isocyanatocyclohexyl)methane, sowie deren Mischungen eingesetzt.

In A2) werden polymere Polyole mit einem zahlenmittleren Molekulargewicht Mn von 400 bis 8000 g/mol, bevorzugt von 400 bis 6000 g/mol und besonders bevorzugt von 600 bis 3000 g/mol eingesetzt. Diese weisen bevorzugt eine OH-Funktionalität von 1,5 bis 6, besonders bevorzugt von 1,8 bis 3, ganz besonders bevorzugt von 1,9 bis 2,1 auf.

Solche polymeren Polyole sind die in der Polyurethanlacktechnologie an sich bekannten Polyesterpolyole, Polyacrylatpolyole, Polyurethanpolyole, Polycarbonatpolyole, Polyetherpolyole, Polyesterpolyacrylatpolyole, Polyurethanpolyacrylatpolyole, Polyurethanpolyesterpolyole, Polyurethanpolyetherpolyole, Polyurethanpolycarbonatpolyole und Polyesterpolycarbonatpolyole. Diese können in A2) einzeln oder in beliebigen Mischungen untereinander eingesetzt werden.

Solche Polyesterpolyole sind die an sich bekannten Polykondensate aus Di- sowie gegebenenfalls Tri-, und Tetraolen und Di- sowie gegebenenfalls Tri- und Tetracarbonsäuren oder Hydroxycarbonsäuren oder Lactonen. Anstelle der freien Polycarbonsäuren können auch die entsprechenden Polycarbonsäureanhydride oder entsprechende Polycarbonsäureester von niederen Alkoholen zur Herstellung der Polyester verwendet werden.

Beispiele für geeignete Diole sind Ethylenglykol, Butylenglykol, Diethylenglykol, Triethylenglykol, Polyalkylenglykole wie Polyethylenglykol, weiterhin 1,2-Propandiol, 1,3-Propandiol, 1,3-Butandiol, 1,4-Butandiol, 1,6-Hexandiol und Isomere, Neopentylglykol oder Hydroxypivalinsäureneopentylglykolester, wobei 1,6-Hexandiol und Isomere, Neopentylglykol und Hydroxypivalinsäureneopenthylglykolester bevorzugt sind. Daneben können auch Polyole wie Trimethylolpropan, Glycerin, Erythrit, Pentaerythrit, Trimethylolbenzol oder Trishydroxyethylisocyanurat eingesetzt werden.

Als Dicarbonsäuren können Phthalsäure, Isophthalsäure, Terephthalsäure, Tetrahydrophthalsäure, Hexahydrophthalsäure, Cyclohexandicarbonsäure, Adipinsäure, Azelainsäure, Sebacinsäure, Glutarsäure, Tetrachlorphthalsäure, Maleinsäure, Fumarsäure, Itaconsäure, Malonsäure, Korksäure, 2-Methylbernsteinsäure, 3,3-Diethylglutarsäure und/oder 2,2-Dimethylbernsteinsäure eingesetzt werden. Als Säurequelle können auch die entsprechenden Anhydride verwendet werden.

Sofern die mittlere Funktionalität des zu veresternden Polyols > als 2 ist, können zusätzlich auch Monocarbonsäuren, wie Benzoesäure und Hexancarbonsäure mit verwendet werden.

Bevorzugte Säuren sind aliphatische oder aromatische Säuren der vorstehend genannten Art. Besonders bevorzugt sind Adipinsäure, Isophthalsäure und gegebenenfalls Trimellithsäure.

Hydroxycarbonsäuren, die als Reaktionsteilnehmer bei der Herstellung eines Polyesterpolyols mit endständigen Hydroxylgruppen mitverwendet werden können, sind beispielsweise Hydroxycapronsäure, Hydroxybuttersäure, Hydroxydecansäure, Hydroxystearinsäure und dergleichen. Geeignete Lactone sind Caprolacton, Butyrolacton und Homologe. Bevorzugt ist Caprolacton.

Ebenfalls können in A2) Hydroxylgruppen aufweisende Polycarbonate, bevorzugt Polycarbonatdiole, mit zahlenmittleren Molekulargewichten Mn von 400 bis 8000 g/mol, bevorzugt 600 bis 3000 g/mol eingesetzt werden. Diese sind durch Reaktion von Kohlensäurederivaten, wie Diphenylcarbonat, Dimethylcarbonat oder Phosgen, mit Polyolen, bevorzugt Diolen, erhältlich.

Beispiele derartiger Diole sind Ethylenglykol, 1,2- und 1,3-Propandiol, 1,3- und 1,4-Butandiol, 1,6-Hexandiol, 1,8-Octandiol, Neopentylglykol, 1,4-Bishydroxymethylcyclohexan, 2-Methyl-1,3-propandiol, 2,2,4-Trimethylpentandiol-1,3, Dipropylenglykol, Polypropylenglykole, Dibutylenglykol, Polybutylenglykole, Bisphenol A und lactonmodifizierte Diole der vorstehend genannten Art.

Bevorzugt enthält das Polycarbonatdiol 40 bis 100 Gew.% Hexandiol, bevorzugt 1,6-Hexandiol und/oder Hexandiolderivate. Solche Hexandiolderivate basieren auf Hexandiol und weisen neben endständigen OH-Gruppen Ester- oder Ethergruppen auf. Solche Derivate sind durch Reaktion von Hexandiol mit überschüssigem Caprolacton oder durch Veretherung von Hexandiol mit sich selbst zum Di- oder Trihexylenglykol erhältlich.

Statt oder zusätzlich zu reinen Polycarbonatdiolen können auch Polyether-Polycarbonatdiole in A2) eingesetzt werden. Die Hydroxylgruppen aufweisenden Polycarbonate sind bevorzugt linear gebaut.

Ebenfalls können in A2) Polyetherpolyole eingesetzt werden. Geeignet sind beispielsweise die in der Polyurethanchemie an sich bekannten Polytetramethylenglykolpolyether wie sie durch Polymerisation von Tetrahydrofuran mittels kationischer Ringöffnung erhältlich sind.

Ebenfalls geeignete Polyetherpolyole sind die an sich bekannten Additionsprodukte von Styroloxid, Ethylenoxid, Propylenoxid, Butylenoxide und/oder Epichlorhydrins an di- oder polyfunktionelle Startermoleküle. Polyetherpolyole, basierend auf der zumindest anteiligen Addition von Ethylenoxid an di- oder polyfunktionelle Startermoleküle, können auch als Komponente A4) eingesetzt werden (nichtionische Hydrophilierungsmittel).

Als geeignete Startermoleküle können alle dem Stand der Technik nach bekannten Verbindungen eingesetzt werden, wie zum Beispiel Wasser, Butyldiglykol, Glycerin, Diethylenglykol, Trimethyolpropan, Propylenglykol, Sorbit, Ethylendiamin, Triethanolamin, 1,4-Butandiol. Bevorzugte Startermoleküle sind Wasser, Ethylenglykol, Propylenglykol, 1,4-Butandiol, Diethylenglykol und Butyldiglykol.

Besonders bevorzugte Ausführungsformen der Polyurethan-Dispersionen (I) enthalten als Komponente A2) eine Mischung aus Polycarbonatpolyolen und Polytetramethylenglykolpolyolen, wobei in dieser Mischung der Anteil an Polycarbonatpolyolen in der Mischung 0 bis 80 Gew.% und der Anteil an Polytetramethylenglykolpolyolen 100 bis 20 Gew.% beträgt. Bevorzugt ist ein Anteil von 50 bis 100 Gew.% an Polytetramethylenglykolpolyolen und ein Anteil von 0 bis 50 Gew.-% an Polycarbonatpolyolen. Besonders bevorzugt ist ein Anteil von 75 bis 100 Gew.% an Polytetramethylenglykolpolyolen und ein Anteil von 0 bis 25 Gew.% an Polycarbonatpolyolen, jeweils mit der Maßgabe, dass die Summe der Gewichtsprozente der Polycarbonat- und Polytetramethylenglykolpolyole 100 % ergibt und der Anteil der Summe der Polycarbonat- und Polytetramethylenglykolpolyetherpolyole an der Komponente A2) mindestens 50 Gew.%, bevorzugt 60 Gew.-% und besonders bevorzugt mindestens 70 Gew.% beträgt.

Die Verbindungen der Komponente A3) besitzen Molekulargewichte von 62 bis 400 g/mol.

In A3) können Polyole des genannten Molekulargewichtsbereichs mit bis zu 20 Kohlenstoffatomen, wie Ethylenglykol, Diethylenglykol, Triethylenglykol, 1,2-Propandiol, 1,3-Propandiol, 1,4-Butandiol, 1,3-Butylenglykol, Cyclohexandiol, 1,4-Cyclohexandimethanol, 1,6-Hexandiol, Neopentylglykol, Hydrochinondihydroxyethylether, Bisphenol A (2,2-Bis(4-hydroxyphenyl)propan), hydriertes Bisphenol A, (2,2-Bis(4-hydroxycyclohexyl)propan), Trimethylolpropan, Glycerin, Pentaerythrit sowie deren beliebige Mischungen untereinander eingesetzt werden.

Geeignet sind auch Esterdiole des genannten Molekulargewichtsbereichs wie α-Hydroxybutyl-ε-hydroxycapronsäureester, ω-Hydroxyhexyl-y-hydroxybuttersäure-ester, Adipinsäure-(β-hydroxyethyl)ester oder Terephthalsäurebis(β-hydroxyethyl)-ester.

Ferner können in A3) auch monofunktionelle, isocyanatreaktive, hydroxylgruppenhaltige Verbindungen eingesetzt werden. Beispiele solcher monofunktionellen Verbindungen sind Ethanol, n-Butanol, Ethylenglykolmonobutylether, Diethylenglykolmonomethylether, Ethylenglykolmonobutylether, Diethylenglykolmonobutylether, Propylenglykolmonomethylether, Dipropylenglykolmonomethylether, Tripropylenglykolmonomethylether, Dipropylenglykolmonopropylether, Propylenglykolmonobutylether, Dipropylenglykolmonobutylether, Tripropylenglykolmonobutylether, 2-Ethylhexanol, 1-Octanol, 1-Dodecanol, 1-Hexadecanol. Bevorzugte Verbindungen der Komponente A3) sind 1,6-Hexandiol. 1,4-Butandiol, Neopentylglykol und Trimethylolpropan.

Unter anionisch bzw. potentiell anionisch hydrophilierenden Verbindungen der Komponente A4) werden sämtliche Verbindungen verstanden, die mindestens eine isocyanatreaktive Gruppe wie eine Hydroxylgruppe aufweisen sowie mindestens eine Funktionalität wie z.B. -COO-M⁺, -SO₃ M⁺, -PO(0⁻M⁺)₂ mit M⁺ beispielsweise gleich Metallkation, H⁺, NH₄⁺, NHR₃⁺, wobei R jeweils ein C1-C12-Alkylrest, C5-C6-Cycloalkylrest und/oder ein C2-C4-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann. Geeignete anionisch oder potentiell anionisch hydrophilierende Verbindungen sind Mono- und Dihydroxycarbonsäuren, Mono - und Dihydroxysulfonsäuren sowie Mono- und Dihydroxyphosphonsäuren und ihre Salze. Beispiele solcher anionischen bzw. potentiell anionischen Hydrophilierungsmittel sind Dimethylolpropionsäure, Dimethylolbuttersäure, Hydroxypivalinsäure, Äpfelsäure, Zitronensäure, Glykolsäure, Milchsäure und das propoxylierte Addukt aus 2-Butendiol und NaHSO₃, wie es in DE-A 2 446 440, Seite 5-9, Formel I-III beschrieben ist. Bevorzugte anionische oder potentiell anionische Hydrophilierungsmittel der Komponente A4) sind solche der vorstehend genannten Art, die über Carboxylat- bzw Carbonsäuregruppen und/oder Sulfonatgruppen verfügen.

Besonders bevorzugte anionische oder potentiell anionische Hydrophilierungsmittel sind solche, die Carboxylat- bzw. Carbonsäuregruppen als ionische oder potentiell ionische Gruppen enthalten, wie Dimethylolpropionsäure, Dimethylolbuttersäure und Hydroxypivalinsäure bzw. deren Salze.

Geeignete nichtionisch hydrophilierende Verbindungen der Komponente A4) sind z.B. Polyoxyalkylenether, die mindestens eine Hydroxy- oder Aminogruppe, bevorzugt mindestens eine Hydroxygruppe enthalten.

Beispiele sind die monohydroxyfunktionellen, im statistischen Mittel 5 bis 70, bevorzugt 7 bis 55 Ethylenoxideinheiten pro Molekül aufweisenden Polyalkylenoxidpolyetheralkohole, wie sie in an sich bekannter Weise durch Alkoxylierung geeigneter Startermoleküle zugänglich sind (z.B. in Ullmanns Encyclopädie der technischen Chemie, 4. Auflage, Band 19, Verlag Chemie, Weinheim S. 31-38).

Diese sind entweder reine Polyethylenoxidether oder gemischte Polyalkylenoxidether, wobei sie mindestens 30 mol%, bevorzugt mindestens 40 mol% bezogen auf alle enthaltenen Alkylenoxideinheiten an Ethylenoxideinheiten enthalten.

Bevorzugte Polyethylenoxidether der vorstehend genannten Art sind monofunktionelle gemischte Polyalkylenoxidpolyether, die 40 bis 100 mol% Ethylenoxid- und 0 bis 60 mol% Propylenoxideinheiten aufweisen.

Bevorzugte nichtionisch hydrophilierende Verbindungen der Komponente A4) sind solche der vorstehend genannten Art, wobei es sich um Block(co)polymere handelt, die durch blockweise Addition von Alkylenoxiden an geeignete Starter hergestellt werden.

Geeignete Startermoleküle für solche nichtionischen Hydrophilierungsmittel sind gesättigte Monoalkohole wie Methanol, Ethanol, n-Propanol, Isopropanol, n-Butanol, Isobutanol, sec-Butanol, die Isomeren Pentanole, Hexanole, Octanole und Nonanole, n-Decanol, n-Dodecanol, n-Tetradecanol, n-Hexadecanol, n-Octadecanol, Cyclohexanol, die isomeren Methylcyclohexanole oder Hydroxymethylcyclohexan, 3-Ethyl-3-hydroxymethyloxetan oder Tetrahydrofurfurylalkohol, Diethylenglykolmonoalkylether, wie beispielsweise Diethylenglykolmonobutylether, ungesättigte Alkohole wie Allylalkohol, 1,1-Dimethylallylalkohol oder Oleinalkohol, aromatische Alkohole wie Phenol, die isomeren Kresole oder Methoxyphenole, araliphatische Alkohole wie Benzylalkohol, Anisalkohol oder Zimtalkohol, sekundäre Monoamine wie Dimethylamin, Diethylamin, Dipropylamin, Diisopropylamin, Dibutylamin, Bis-(2-ethylhexyl)-amin, N-Methyl- und N-Ethylcyclohexylamin oder Dicyclohexylamin sowie heterocyclische sekundäre Amine wie Morpholin, Pyrrolidin, Piperidin oder 1H-Pyrazol. Bevorzugte Startermoleküle sind gesättigte Monoalkohole der vorstehend genannten Art. Besonders bevorzugt werden Diethylenglykolmonobutylether oder n-Butanol als Startermoleküle verwendet.

Für die Alkoxylierungsreaktion geeignete Alkylenoxide sind insbesondere Ethylenoxid und Propylenoxid, die in beliebiger Reihenfolge oder auch im Gemisch bei der Alkoxylierungsreaktion eingesetzt werden können.

Als Komponente B1) können Di- oder Polyamine wie 1,2-Ethylendiamin, 1,2- und 1,3-Diaminopropan, 1,4-Diaminobutan, 1,6-Diaminohexan, Isophorondiamin, Isomerengemisch von 2,2,4- und 2,4,4-Trimethylhexamethylendiamin, 2-Methylpentamethylendiamin, Diethylentriamin, Triaminononan, 1,3- und 1,4-Xylylendiamin, α,α,α',α'-Tetramethyl-1,3- und -1,4-xylylendiamin und 4,4-Diaminodicyclohexylmethan und/oder Dimethylethylendiamin eingesetzt werden. Ebenfalls möglich, aber weniger bevorzugt, ist die Verwendung von Hydrazin sowie Hydraziden wie Adipinsäuredihydrazid.

Darüber hinaus können als Komponente B1) auch Verbindungen, die neben einer primären Aminogruppe auch sekundäre Aminogruppen oder neben einer Aminogruppe (primär oder sekundär) auch OH-Gruppen aufweisen, eingesetzt werden. Beispiele hierfür sind primäre/sekundäre Amine, wie Diethanolamin, 3-Amino-1-methylaminopropan, 3-Amino-1-ethylaminopropan, 3-Amino-1-cyclohexylaminopropan, 3-Amino-1-Methylaminobutan, Alkanolamine wie N-Aminoethylethanolamin, Ethanolamin, 3-Aminopropanol, Neopentanolamin.

Ferner können als Komponente B1) auch monofunktionelle isocyanatreaktive Aminverbindungen eingesetzt werden, wie beispielsweise Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketime von diprimären Aminen, primär/tertiäre Amine, wie N,N-Dimethylaminopropylamin.

Bevorzugte Verbindungen der Komponente B1) sind 1,2-Ethylendiamin, 1,4-Diaminobutan und Isophorondiamin. Besonders bevorzugt werden Mischungen der vorgenannten Diamine der Komponente B1) eingesetzt, insbesondere Mischungen aus 1,2-Ethylendiamin und Isophorondiamin sowie Mischungen aus 1,4-Diaminobutan und Isophorondiamin.

Unter anionisch bzw. potentiell anionisch hydrophilierenden Verbindungen der Komponente B2) werden sämtliche Verbindungen verstanden, die mindestens eine isocyanatreaktive Gruppe, bevorzugt eine Amino-Gruppe aufweisen, sowie mindestens eine Funktionalität wie z.B. -COO⁻M⁺, -SO₃⁻M⁺, -PO(O⁻M⁺)₂ mit M⁺ beispielsweise gleich Metallkation, H⁺, NH₄⁺, NHR₃⁺, wobei R jeweils ein C1-C12-Alkylrest, C5-C6-Cycloalkylrest und/oder ein C2-C4-Hydroxyalkylrest sein kann, die bei Wechselwirkung mit wässrigen Medien ein pH-Wert-abhängiges Dissoziationsgleichgewicht eingeht und auf diese Weise negativ oder neutral geladen sein kann.

Geeignete anionisch oder potentiell anionisch hydrophilierende Verbindungen sind Mono- und Diaminocarbonsäuren, Mono- und Diaminosulfonsäuren sowie Mono- und Diaminophosphonsäuren und ihre Salze. Beispiele solcher anionischen bzw. potentiell anionischen Hydrophilierungsmittel sind N-(2-Aminoethyl)-β-alanin, 2-(2-Amino-ethylamino)-ethansulfonsäure, Ethylendiaminpropyl- oder -butylsulfonsäure, 1,2- oder 1,3-Propylendiamin-β-ethylsulfonsäure, Glycin, Alanin, Taurin, Lysin, 3,5-Diaminobenzoesäure und das Additionsprodukt von IPDA und Acrylsäure (EP-A 0 916 647, Beispiel 1). Weiterhin kann Cyclohexylaminopropansulfonsäure (CAPS) aus WO-A 01/88006 als anionisches oder potentiell anionisches Hydrophilierungsmittel verwendet werden.

Bevorzugte anionische oder potentiell anionische Hydrophilierungsmittel der Komponente B2) sind solche der vorstehend genannten Art, die über Carboxylat- bzw. Carobonsäuregruppen und/oder Sulfonatgruppen verfügen wie die Salze von N-(2-Aminoethyl)-β-alanin, der 2-(2-Aminoethylamino)ethansulfonsäure oder des Additionsproduktes von IPDA und Acrylsäure (EP-A 0 916 647, Beispiel 1).

Zur Hydrophilierung können auch Mischungen aus anionischen bzw. potentiell anionischen Hydrophilierungsmitteln und nichtionischen Hydrophilierungsmitteln verwendet werden.

In einer bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.-% addieren:
5 bis 40 Gew.% Komponente A1),
55 bis 90 Gew.% A2),
0 bis 20 Gew.% Summe der Komponenten A3) und B1)
0,1 bis 25 Gew.% Summe der Komponenten A4) und B2), wobei bezogen auf die Gesamtmengen der Komponenten A1) bis A4) und B1) bis B2) 0,1 bis 10 Gew.% an anionischen bzw. potentiell anionischen Hydrophilierungsmitteln aus A4) und/oder B2) verwendet werden.

In einer besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.% aufaddieren:
5 bis 35 Gew.% Komponente A1),
60 bis 90 Gew.% A2),
0,5 bis 15 Gew.% Summe der Komponenten A3) und B1)
0,5 bis 15 Gew.% Summe der Komponenten A4) und B2), wobei bezogen auf die Gesamtmengen der Komponenten A1) bis A4) und B1) bis B2) 0,5 bis 7 Gew.% an anionischen bzw. potentiell anionischen Hydrophilierungsmitteln aus A4) und/oder B2) verwendet werden.

In einer ganz besonders bevorzugten Ausführungsform zur Herstellung der speziellen Polyurethan-Dispersionen werden die Komponenten A1) bis A4) und B1) bis B2) in den folgenden Mengen eingesetzt, wobei sich die Einzelmengen stets zu 100 Gew.% aufaddieren:
10 bis 30 Gew.% Komponente A1),
65 bis 85 Gew.% A2),
0,5 bis 14 Gew.% Summe der Komponenten A3) und B1)
1 bis 10 Gew.% Summe der Komponenten A4) und B2), wobei bezogen auf die Gesamtmengen der Komponenten A1) bis A4) und B1) bis B2) 1 bis 5 Gew.% an anionischen bzw. potentiell anionischen Hydrophilierungsmitteln aus A4) und/oder B2) und einem Anteil von 1 bis 5 Gew.-%, bevorzugt weniger als 1,0 Gew.%, besonders bevorzugt weniger als 0,5 Gew.% und ganz besonders bevorzugt keine nicht-ionisch hydrophilierenden Bausteinen verwendet werden.

Die Herstellung der anionisch hydrophilierten Polyurethan-Dispersionen (I) kann in einer oder mehreren Stufe/-n in homogener oder bei mehrstufiger Umsetzung, teilweise in disperser Phase durchgeführt werden. Nach vollständig oder teilweise durchgeführter Polyaddition aus A1) bis A4) erfolgt ein Dispergier-, Emulgier- oder Lösungsschritt. Im Anschluss erfolgt gegebenenfalls eine weitere Polyaddition oder Modifikation in disperser Phase.

Dabei können alle aus dem Stand der Technik bekannten Verfahren wie z.B. Prepolymer-Mischverfahren, Acetonverfahren oder Schmelzdispergierverfahren verwendet werden. Bevorzugt wird das Aceton-Verfahren verwendet.

Für die Herstellung nach dem Aceton-Verfahren werden üblicherweise die Bestandteile A2) bis A4) und die Polyisocyanatkomponente A1) zur Herstellung eines isocyanatfunktionellen Polyurethan-Prepolymers ganz oder teilweise vorgelegt und gegebenenfalls mit einem mit Wasser mischbaren aber gegenüber Isocyanatgruppen inerten Lösungsmittel verdünnt und auf Temperaturen im Bereich von 50 bis 120°C aufgeheizt. Zur Beschleunigung der Isocyanatadditionsreaktion können die in der Polyurethan-Chemie bekannten Katalysatoren eingesetzt werden.

Geeignete Lösungsmittel sind die üblichen aliphatischen, ketofunktionellen Lösemittel wie Aceton, 2-Butanon, die nicht nur zu Beginn der Herstellung, sondern gegebenenfalls in Teilen auch später zugegeben werden können. Bevorzugt sind Aceton und 2-Butanon.

Andere Lösemittel wie Xylol, Toluol, Cyclohexan, Butylacetat, Methoxypropylacetat, N-Methylpyrrolidon, N-Ethylpyrrolidon, Lösemittel mit Ether- oder Estereinheiten können zusätzlich eingesetzt und ganz oder teilweise abdestilliert werden oder vollständig im Falle von N-Methylpyrrolidon, N-Ethylpyrrolidon in der Dispersion verbleiben. Bevorzugt werden aber außer den genannten aliphatischen, ketofunktionellen Lösemitteln keine anderen Lösungsmittel verwendet.

Anschließend werden die gegebenenfalls zu Beginn der Reaktion noch nicht zugegebenen Bestandteile von A1) bis A4) zudosiert.

Bei der Herstellung des Polyurethan-Prepolymeren aus A1) bis A4) beträgt das Stoffmengenverhältnis von Isocyanatgruppen zu mit isocyanatreaktiven Gruppen 1,05 bis 3,5; bevorzugt 1,2 bis 3,0 und besonders bevorzugt 1,3 bis 2,5.

Die Umsetzung der Komponenten A1) bis A4) zum Prepolymer erfolgt teilweise oder vollständig, bevorzugt aber vollständig. Es werden so Poiyurethan-Prepolymere, die freie Isocyanatgruppen enthalten, in Substanz oder in Lösung erhalten.

Im Neutralisationsschritt zur teilweisen oder vollständigen Überführung potentiell anionischer Gruppen in anionische Gruppen werden Basen wie tertiäre Amine, z.B. Trialkylamine mit 1 bis 12, bevorzugt 1 bis 6 C-Atomen, besonders bevorzugt 2 bis 3 C-Atomen in jedem Alkylrest oder Alkalimetallbasen wie die entsprechenden Hydroxide eingesetzt.

Beispiele hierfür sind Trimethylamin, Triethylamin, Methyldiethylamin, Tripropylamin, N-Methylmorpholin, Methyldiisopropylamin, Ethyldiisopropylamin und Diisopropylethylamin. Die Alkylreste können beispielsweise auch Hydroxylgruppen tragen, wie bei den Dialkylmonoalkanol-, Alkyldialkanol- und Trialkanolaminen. Als Neutralisationsmittel sind gegebenenfalls auch anorganische Basen, wie wässrige Ammoniaklösung oder Natrium- bzw. Kaliumhydroxid einsetzbar.

Bevorzugt sind Ammoniak, Triethylamin, Triethanolamin, Dimethylethanolamin oder Diisopropylethylamin sowie Natriumhydroxid und Kaliumhydroxid, besonders bevorzugt sind Natriumhydroxid und Kaliumhydroxid.

Die Stoffmenge der Basen beträgt zwischen 50 und 125 mol%, bevorzugt zwischen 70 und 100 mol% der Stoffmenge der zu neutralisierenden Säuregruppen. Die Neutralisation kann auch gleichzeitig mit der Dispergierung erfolgen, indem das Dispergierwasser bereits das Neutralisationsmittel enthält.

Im Anschluss wird in einem weiteren Verfahrensschritt, falls noch nicht oder nur teilweise geschehen das erhaltene Prepolymer mit Hilfe von aliphatischen Ketonen wie Aceton oder 2-Butanon gelöst.

Bei der Kettenverlängerung in Stufe B) werden NH₂- und/oder NH-funktionelle Komponenten mit den noch verbliebenen Isocyanatgruppen des Prepolymers teilweise oder vollständig umgesetzt. Bevorzugt wird die Kettenverlängerung/-terminierung vor der Dispergierung in Wasser durchgeführt.

Zur Kettenterminierung werden üblicherweise Amine B1) mit einer gegenüber Isocyanaten reaktiven Gruppe wie Methylamin, Ethylamin, Propylamin, Butylamin, Octylamin, Laurylamin, Stearylamin, Isononyloxypropylamin, Dimethylamin, Diethylamin, Dipropylamin, Dibutylamin, N-Methylaminopropylamin, Diethyl(methyl)aminopropylamin, Morpholin, Piperidin, bzw. geeignete substituierte Derivate davon, Amidamine aus diprimären Aminen und Monocarbonsäuren, Monoketime von diprimären Aminen, primär/tertiäre Amine, wie N,N-Dimethylaminopropylamin verwendet.

Werden zur teilweisen oder vollständigen Kettenverlängerung anionische oder potentiell anionische Hydrophilierungsmittel entsprechend der Definition B2) mit NH₂- oder NH-Gruppen eingesetzt, erfolgt die Kettenverlängerung der Prepolymere bevorzugt vor der Dispergierung.

Die aminischen Komponenten B1) und B2) können gegebenenfalls in wasser- oder lösemittelverdünnter Form im erfindungsgemäßen Verfahren einzeln oder in Mischungen eingesetzt werden, wobei grundsätzlich jede Reihenfolge der Zugabe möglich ist.

Wenn Wasser oder organische Lösemittel als Verdünnungsmittel mit verwendet werden, so beträgt der Verdünnungsmittelgehalt in der in B) eingesetzten Komponente zur Kettenverlängerung bevorzugt 70 bis 95 Gew.-%.

Die Dispergierung erfolgt bevorzugt im Anschluss an die Kettenverlängerung. Dazu wird das gelöste und kettenverlängerte Polyurethanpolymer gegebenenfalls unter starker Scherung, wie z.B. starkem Rühren, entweder in das Dispergierwasser eingetragen oder es wird umgekehrt das Dispergierwasser zu den kettenverlängerte Polyurethanpolymerlösungen gerührt. Bevorzugt wird das Wasser in das gelöste kettenverlängerte Polyurethanpolymer gegeben.

Das in den Dispersionen nach dem Dispergierschritt noch enthaltene Lösemittel wird üblicherweise anschließend destillativ entfernt. Eine Entfernung bereits während der Dispergierung ist ebenfalls möglich.

Der Restgehalt an organischen Lösemitteln in den Polyurethan-Dispersionen (I) beträgt typischerweise weniger als 1,0 Gew.%, bevorzugt weniger als 0,5 Gew.%, bezogen auf die gesamte Dispersion.

Der pH-Wert der erfindungswesentlichen Polyurethan-Dispersionen (I) beträgt typischerweise weniger als 9,0, bevorzugt weniger als 8,5, besonders bevorzugt weniger als 8,0 und liegt ganz besonders bevorzugt bei 6,0 bis 7,5.

Der Feststoffgehalt der Polyurethan-Dispersionen (I) beträgt bevorzugt 35 bis 70 Gew.%, besonders bevorzugt 40 bis 65 Gew.%, ganz besonders bevorzugt 45 bis 60 Gew.% und insbesondere 45 bis 55 Gew.-%.

Die Menge an anionischen oder potentiell anionischen Gruppen auf der Partikeloberfläche, gemessen über eine Säure-Base-Titration liegt im Allgemeinen bei 2 bis 500 mmol, bevorzugt 30 bis 400 mmol pro 100 Gramm Festkörper.

Die Polyurethandispersionen (I) können nichtfunktionell oder über Hydroxyl- oder Aminogruppen funktionalisiert sein. Darüber hinaus können die Dispersionen (I) in einer nicht bevorzugten Ausführungsform auch über reaktive Gruppen in Form von blockierten Isocyanatgruppen, wie beispielsweise in der DE-A 19 856 412 beschrieben, verfügen.

Die in den erfindungswesentlichen Zusammensetzungen enthaltenen wässrigen, kationisch oder potentiell kationisch hydrophilierten Polyurethan-Dispersionen (II) sind erhältlich, indem
C) isocyanatfunktionelle Prepolymere aus
   C1) organischen Polyisocyanaten wie sie in A1) beschrieben sind
   C2) den in A2) genannten polymeren Polyolen
   C3) gegebenenfalls hydroxyfunktionellen Verbindungen wie sie in A3) beschrieben sind und
   C4) gegebenenfalls isocyanatreaktiven Verbindungen, die kationische Gruppen oder in kationische Gruppen überführbare Einheiten enthalten und/oder gegebenenfalls die in A4) genannten nichtionisch hydrophilierenden Verbindungen enthalten
   hergestellt werden,
D) dessen freie NCO-Gruppen dann ganz oder teilweise
   D1) gegebenenfalls mit den in B1) beschriebenen aminofunktionellen Verbindungen und
   D2) gegebenenfalls mit isocyanatreaktiven, bevorzugt aminofunktionellen, kationischen oder potentiell kationischen Hydrophilierungsmitteln
unter Kettenverlängerung umgesetzt werden und die Prepolymere vor während oder nach Schritt D) in Wasser dispergiert werden, wobei gegebenenfalls enthaltene potentiell ionische Gruppen durch teilweise oder vollständige Umsetzung mit einem Neutralisationsmittel in die ionische Form überführt werden.

Bevorzugte wässrige, kationische Polyurethan-Dispersionen (II) haben einen niedrigen Grad an hydrophilen kationischen Gruppen, bevorzugt von 2 bis 200 Milliäquivalenten, besonders bevorzugt 10 bis 100 Milliäquivalenten pro 100 g Festharz.

Um eine kationische Hydrophilierung zu erreichen, müssen in C4) und/oder D2) Hydrophilierungsmittel eingesetzt werden, die wenigstens eine gegenüber NCO-Gruppen reaktive Gruppe aufweisen und darüber hinaus kationische Gruppen oder in kationische Gruppen überführbare Einheiten enthalten. Beispiele für Isocyanat-reaktive Gruppen sind Thiol- und Hydroxlgruppen, als primäre oder sekundäre Amine eignen sich beliebige hydroxy- und/oder aminofunktionelle mono - und insbesondere bifunktionelle Verbindungen mit mindestens einem tertiären Aminstickstoffatom, deren tertiäre Stickstoffatome während oder nach Beendigung der Isocyanat-Polyadditionsreaktion durch Neutralisierung oder Quaternisierung zumindest teilweise in quartäre Ammoniumgruppen überführt werden können. Hierzu gehören beispielsweise Verbindungen wie 2-(N,N-Dimethylamino)-ethylamin, N-Methyl-diethanolamin, N-Methyl-diisopropanolamin, N-Ethyl-diethanolamin, N-Ethyl-diisopropanolamin, N,N'-Bis-(2-hydroxyethyl)-perhydropyrazin, N-Methyl-bis(3-aminopropyl)-amin, N-Methyl-bis(2-aminoethyl)-amin, N,N'-, N"-Trimethyldiethylentriamin, N,N-Dimethylaminoethanol, N,N-Diethylaminoethanol, 1-N,N-Diethylamino-2-aminoethan, 1-N,N-Diethylamino-3-aminopropan, 2-Dimethylaminomethyl-2-methyl-propandiol-1,3, Triethanolamin, Tripropanolamin, Triisopropanolamin, N-Isopropyl-diethanolamin, N-Butyldiethanolamin, N-Isobutyl-diethanolamin, N-Oleyl-diethanolamin, N-Stearyldiethanolamin, o-xethyliertes Kokosfettamin, N-Allyl-diethanolamin, N-Methyl-diisopropanolamin, N,N-Propyldiisopropanolamin, N-Butyl-diisopropanolamin und/oder N-Cyclohexyl-diisopropanolamin. Auch der Einbau von tertiären und quaternären Ammoniumgruppen nebeneinander oder der Einbau von Mischungen der genannten aminofunktionellen Hydrophilierungsmitteln ist möglich.

Zur Generierung der kationischen Hydrophilierung erfolgt der Einbau der ionischen Gruppen, d.h. der ternären bzw. quaternären Ammoniumgruppen, vorzugsweise unter Mitverwendung von tertiäre Aminogruppen aufweisenden Aufbaukomponenten unter anschließender Überführung der tertiären Aminogruppen in die entsprechenden Ammoniumgruppen durch Neutralisation mit anorganischen oder organischen Säuren wie z.B. Phosphorsäure, Salzsäure, Essigsäure, Fumarsäure, Adipinsäure, Maleinsäure, Milchsäure, Weinsäure, Oxalsäure, Äpfelsäure, Zitronensäure, Ascorbinsäure oder N-Methyl-N-(methylaminocarbonyl)-aminomethansulfonsäure oder durch Quatemierung mit geeigneten Quaternierungsmitteln wie z.B. Methylchlorid, Methyljodid, Dimethylsulfat, Benzylchlorid, Chloressigsäureethylester oder Bromacetamid. Grundsätzlich kann diese Neutralisation oder Quaternierung der tertiären Stickstoff aufweisenden Aufbaukomponenten auch vor oder während der Isocyanat-Polyadditionsreaktion erfolgen, obwohl dies weniger bevorzugt ist. Es ist auch möglich, ternäre bzw. quaternäre Ammoniumgruppen in die Polyisocyanat-Polyadditionsprodukte über tertiäre Aminogruppen aufweisende, als eingesetzte Polyetherpolyole unter anschließender Neutralisation bzw. Quaternierung der tertiären Aminogruppen einzuführen. Auch der Einbau von quaternären Ammoniumgruppen und von tertiären Aminogruppen nebeneinander oder Mischungen ist möglich.

Die Neutralisation kann auch gleichzeitig mit der Dispergierung in Wasser erfolgen, beispielsweise durch Lösen des Neutralisationsmittels in Wasser, parallele Zugabe des Neutralisationsmittels und des Wassers oder durch Zugabe des Neutralisationsmittels nach der Zugabe des Wassers.

Der Neutralisations- oder Quaternierungsgrad als Äquivalentsverhältnis von Säureprotonen bzw. Quarternisierungsmittel zu potentiell kationischen Gruppen der Komponenten C4 und/oder D2 wird im Allgemeinen zwischen 20 und 300%, bevorzugt 50 bis 200% und besonders bevorzugt zwischen 70 und 130% eingestellt.

Die Herstellung der kationisch hydrophilierten Polyurethan-Dispersionen (II) erfolgt analog den für anionisch hydrophilierten Polyurethan-Dispersionen (I) beschriebenen Prinzipien und Methoden.

Der Restgehalt an organischen Lösemitteln in den Polyurethan-Dispersionen (II) beträgt ebenso typischerweise weniger als 1,0 Gew.%, bevorzugt weniger als 0,5 Gew.%, bezogen auf die gesamte Dispersion.

Der pH-Wert der erfindungswesentlichen Polyurethan-Dispersionen (II) beträgt typischerweise 2 bis 8, bevorzugt weniger als 7, besonders bevorzugt weniger als 6 und liegt ganz besonders bevorzugt bei 4 bis 6.

In einer bevorzugten Ausführungsform zur Herstellung der kationisch hydrophilierten Polyurethan-Dispersionen (II) werden die Komponenten C1) bis C4) und D1) bis D2) in den folgenden Mengen eingesetzt, wobei sich die Einzelmengen stets zu 100 Gew.% addieren:
5 bis 40 Gew.% Komponente C1),
55 bis 90 Gew.% Komponente C2),
0,1 bis 20 Gew.% Summe der Komponenten C3) und D1),
0,1 bis 30 Gew.% Summe der Komponenten C4) und D2), wobei bezogen auf die Gesamtmengen der Komponenten C1) bis C4) und D1) bis D2) 0,1 bis 20 Gew.% an kationischen bzw. potentiell kationischen Hydrophilierungsmitteln aus C4) und/oder D2) verwendet werden.

In einer besonders bevorzugten Ausführungsform zur Herstellung der kationisch hydrophilierten Polyurethan-Dispersionen (II) werden die Komponenten C1) bis C4) und D1) bis D2) in den folgenden Mengen eingesetzt, wobei sich die Einzelmengen stets zu 100 Gew.% addieren:
10 bis 40 Gew.% Komponente C1),
65 bis 90 Gew.% Komponente C2),
0,1 bis 15 Gew.% Summe der Komponenten C3) und D1),
1 bis 25 Gew.% Summe der Komponenten C4) und D2), wobei bezogen auf die Gesamtmengen der Komponenten C1) bis C4) und D1) bis D2) 1 bis 15 Gew.% an kationischen bzw. potentiell kationischen Hydrophilierungsmitteln aus C4) und/oder D2) verwendet werden.

In einer ganz besonders bevorzugten Ausführungsform zur Herstellung der kationisch hydrophilierten Polyurethan-Dispersionen (II) werden die Komponenten C1) bis C4) und D1) bis D2) in den folgenden Mengen eingesetzt, wobei sich die Einzelmengen stets zu 100 Gew.% addieren:
10 bis 30 Gew.-% Komponente C1),
65 bis 85 Gew.-% Komponente C2),
0,5 bis 10 Gew.-% Summe der Komponenten C3) und D1),
1 bis 15 Gew.-% Summe der Komponenten C4) und D2), wobei bezogen auf die Gesamtmengen der Komponenten C1) bis C4) und D1) bis D2) 1 bis 10 Gew.-% an kationischen bzw. potentiell kationischen Hydrophilierungsmitteln aus C4) und/oder D2) verwendet werden.

Der Anteil an nicht-ionisch hydrophilierenden Bausteinen liegt bevorzugt bei weniger als 20 Gew.-%, besonders bevorzugt bei weniger als 8 Gew.-% und ganz besonders bevorzugt bei weniger als 3 Gew.-%. Insbesondere sind keine nicht-ionisch hydrophilierenden Bausteine enthalten.

Die kationisch hydrophilierte Polyurethan-Dispersionen (II) weisen im Allgemeinen einen Festkörpergehalt von 10 bis 65 Gew.-%, bevorzugt von 20 bis 55 Gew.-%, besonders bevorzugt von 25 bis 45 Gew.-% auf.

Bevorzugte kationisch, hydrophilierte Polyurethan-Dispersionen (II) enthalten Partikel mit einer Partikelgröße bis 800 nm, bevorzugt von 20 bis 500 nm.

Die Menge an kationischen oder potentiell kationischen Gruppen auf der Partikeloberfläche, gemessen über eine Säure-Base-Titration liegt im Allgemeinen zwischen 2 bis 500 mmol, bevorzugt von 30 bis 400 mmol pro 100 Gramm Festkörper.

Neben den Polyurethan-Dispersionen (I) und (II) können auch Hilfs- und Zusatzstoffe (III) mitverwendet werden.

Beispiele für solche Hilfs- und Zusatzstoffe (III) sind Schaumhilfsmittel wie Schaumbildner und -stabilisatoren, Verdicker bzw. Thixotropiermittel, Antioxidantien, Lichtschutzmittel, Emulgatoren, Weichmacher, Pigmente, Füllstoffe, Additive zur Gebindestabilisierung, Biozide, pH-Regulatoren, Dispersionen und/oder Verlaufshilfsmittel. Je nach gewünschtem Eigenschaftsbild und Verwendungszweck der erfindungsgemäßen Beschichtungsmittel auf PUR-Dispersionsbasis können bis zu 70 Gew.-%, bezogen auf Gesamttrockensubstanz, solcher Füllstoffe im Endprodukt enthalten sein.

Bevorzugt sind als Hilfs- und Zusatzstoffe (III) Schaumhilfsmittel wie Schaumbildner und -stabilisatoren enthalten. Geeignet sind beispielsweise handelsübliche Verbindungen wie Fettsäureamide, Sulfosuccinamide Kohlenwasserstoffsulfonate, -sulfate oder Fettsäuresalze, wobei der lipophile Rest bevorzugt 12 bis 24 Kohlenstoffatome enthält, sowie Alkylpolyglycoside, die nach dem Fachmann an sich bekannten Methoden durch Umsetzung von längerkettigen Monoalkoholen (4 bis 22 C-Atome im Alkylrest) mit Mono-, Di- oder Polysacchariden erhältlich sind (siehe z.B. Kirk-Othmer, Encyclopedia of Chemical Technology, John Wiley & Sons, Vol. 24, S. 29). Besonders geeignete Schaumhilfsmittel sind EO/PO-Blockcopolymere, die nach dem Fachmann an sich bekannten Methoden durch Addition von Ethylenoxid und Propylenoxid an OH- oder NH-funktionelle Startermoleküle erhältlich sind (siehe z.B. Kirk-Othmer, Encyclopedia of Chemical Technology, John Wiley & Sons, Vol. 24, S. 28). Zur Verbesserung der Schaumbildung, Schaumstabilität oder der Eigenschaften des resultierenden Polyurethan-Schaums, können neben den EO/PO-Blockcopolymeren noch weitere Additive in der Komponente (III) enthalten sein. Solche weiteren Additive können grundsätzlich alle an sich bekannten anionischen, nichtionischen und kationischen Tenside sein. Bevorzugt werden jedoch allein die EO/PO-Blockcopolymere als Komponente (III) eingesetzt.

Als Verdicker können handelsübliche Verdicker eingesetzt werden, wie Dextrin-, Stärke-, Polysaccharidderivate wie Guargummi oder Cellulosederivate wie Celluloseether oder Hydroxyethylcellulose, organische vollsynthetische Verdicker auf Basis von Polyacrylsäuren, Polyvinylpyrrolidonen, Poly(meth)acrylverbindungen oder Polyurethanen (assoziative Verdicker) sowie anorganische Verdicker, wie Betonite oder Kieselsäuren.

Ebenfalls möglich ist die Zugabe, Einarbeitung oder Beschichtung von bzw. mit antimikrobiellen oder biologischen Wirkstoffen, welche sich beispielsweise in Bezug auf die Wundheilung und die Vermeidung von Keimbelastungen positiv auswirken.

Bevorzugte Wirkstoffe der vorgenannten Art sind solche aus der Gruppe der Antiseptika, Wachstumsfaktoren, Proteaseinhibitoren und nichtsteroidalen Antiphlogistika/Opiate oder auch Wirkstoffe wie beispielsweise Thrombin alfa zur lokalen Blutstillung.

In einer bevorzugten Ausführungsform der vorliegenden Erfindung umfasst der Wirkstoff zumindest ein Bakteriostatikum oder ein Bakterizid, ganz besonders bevorzugt ein antiseptisches Biguanid und/oder dessen Salz, vorzugsweise das Hydrochlorid.

Als Biguanide werden Verbindungen bezeichnet, die sich vom Biguanid (C₂H₇N₅) ableiten, insbesondere dessen Polymere. Antiseptische Biguanide sind solche Verbindungen, die eine antimikrobielle Wirkung aufweisen, also als Bakteriostatika oder vorzugsweise als Bakterizide wirken. Die Verbindungen weisen vorzugsweise eine breite Wirkung gegen viele Bakterien auf und können durch eine Wirkung gegen E. coli von mindestens 0,5 µg/ml, vorzugsweise mindestens 12 oder mindestens 25 µg/ml gekennzeichnet werden (minimal mikrobizide Konzentration oder MMK, gemessen im Suspensionsversuch).

Ein bevorzugtes antiseptisches Biguanid gemäß dieser Erfindung ist Poly(imino[iminocarbonyl]iminopolymethylen), wobei die Verwendung von Poly(hexamethylen)biguanid (PHMB), das auch als Polyhexanid bezeichnet wird, als antiseptisches Biguanid besonders bevorzugt ist.

Der Begriff "antiseptische Biguanide" gemäß dieser Erfindung beinhaltet auch Metaboliten und/oder Prodrugs der antiseptischen Biguaniden. Antiseptische Biguanide können dabei als Racemate oder reine Isoformen vorliegen.

Bevorzugt enthalten die geschäumten Artikel aus Polyurethan-Schäumen beziehungsweise die Zusammensetzungen gemäß der vorliegenden Erfindung antiseptisches Biguanid und/oder dessen Salz, vorzugsweise das Hydrochlorid, in einer Konzentration von 0,010 bis 20 Gew-%, wobei die Konzentration von 0,1 bis 5 Gew-% besonders vorteilhaft ist. Das Biguanid kann eine beliebige Molekulargewichtsverteilung aufweisen.

Grundsätzlich können, wenn auch nicht bevorzugt, die erfindungswesentlichen Zusammensetzungen auch Vernetzer wie unblockierte Polyisocyanate, Amid- und Amin-Formaldehydharze, Phenolharze, Polyaziridine, Aldehyd- und Ketonharze, wie z.B. Phenol-Formaldehydharze, Resole, Furanharze, Harnstoffharze, Carbamidsäureesterharze, Triazinharze, Melaminharze, Benzoguanaminharze, Cyanamidharze oder Anilinharze enthalten.

Beispiele für erfindungsgemäße Zusammensetzungen werden nachfolgend aufgeführt, wobei die Summe der Angaben in Gewichts-% einen Wert von ≤ 100 Gewichts-% einnimmt. Diese Zusammensetzungen umfassen, bezogen auf die Trockensubstanz, typischerweise ≥80 Gewichtsteile bis ≤ 100 Gewichtsteile der Dispersionen (I) und (II) in Summe, ≥ 0 Gewichtsteile bis ≤ 10 Gewichtsteile Schaumhilfsmittel, ≥ 0 Gewichtsteile bis ≤ 10 Gewichtsteile Vernetzer und ≥0 Gewichtsteile bis ≤10 Gewichtsteile Verdicker.

Bevorzugt umfassen diese erfindungsgemäßen Zusammensetzungen, bezogen auf die Trockensubstanz, ≥85 Gewichtsteile bis ≤100 Gewichtsteile der Dispersionen (I) und (II) in Summe, ≥0 Gewichtsteile bis ≤7 Gewichtsteile Schaumhilfsmittel, ≥0 Gewichtsteile bis ≤5 Gewichtsteile Vernetzer, ≥0 Gewichtsteile bis ≤10 Gewichtsteile Antiseptika bzw. Biozide und ≥0 Gewichtsteile bis ≤5 Gewichtsteile Verdicker.

Besonders bevorzugt umfassen diese erfindungsgemäßen Zusammensetzungen, bezogen auf die Trockensubstanz, ≥ 89 Gewichtsteile bis ≤100 Gewichtsteile der Dispersionen (I) und (II) in Summe, ≥0 Gewichtsteile bis ≤6 Gewichtsteile Schaumhilfsmittel, ≥0 Gewichtsteile bis ≤4 Gewichtsteile Vernetzer und ≥0 Gewichtsteile bis ≤4 Gewichtsteile Verdicker.

Bevorzugt besitzt die ggf. mit Komponenten (III) abgemischte Komponente (I) bzw. Komponente (II) eine Viskosität von ≤1000 mPa·s, besonders bevorzugt ≤700 mPa·s, ganz besonders bevorzugt von ≤00 mPa·s und insbesondere von ≤200 mPa·s.

Beispiele für erfindungsgemäße Zusammensetzungen, welche Ethylenoxid/Propylenoxid-Blockcopolymere als Schaumstabilisatoren umfassen, werden nachfolgend aufgeführt. Diese Zusammensetzungen umfassen, bezogen auf Trockensubstanz, ≥80 Gewichtsteile bis ≤100 Gewichtsteile der Dispersionen (I) und (II) in Summe und ≥0 Gewichtsteile bis ≤20 Gewichtsteile der Ethylenoxid/Propylenoxid-Blockcopolymere. Bevorzugt umfassen die Zusammensetzungen, bezogen auf Trockensubstanz, ≥85 Gewichtsteile bis ≤100 Gewichtsteile der Dispersionen (I) und (II) in Summe und ≥0 bis ≤15 Gewichtsteile der Ethylenoxid/Propylenoxid-Blockcopolymere. Besonders bevorzugt sind hierbei ≥90 Gewichtsteile bis ≤100 Gewichtsteile der Dispersionen (I) und (II) in Summe und ≥0 Gewichtsteile bis ≤10 Gewichtsteile der Ethylenoxid/Propylenoxid-Blockcopolymere und ganz besonders bevorzugt ≥94 Gewichtsteile bis ≤100 Gewichtsteile der Dispersionen (I) und (II) in Summe und ≥0 bis ≤6 Gewichtsteile der Ethylenoxid/Propylenoxid-Blockcopolymere.

Im Sinne der vorliegenden Erfindung bedeutet die Angabe "Gewichtsteile" einen relativen Anteil, jedoch nicht im Sinne der Angabe von Gewichts-%. Folglich kann die zahlenmäßige Summe der Gewichtsanteile auch Werte unter bzw. über 100 annehmen.

Neben den genannten Komponenten (I), (II) und (III) können in den erfindungsgemäßen Zusammensetzungen auch weitere wässrige Bindemittel eingesetzt werden. Solche wässrigen Bindemittel können zum Beispiel aus Polyester-, Polyacrylat-, Polyepoxid- oder anderen Polyurethanpolymeren aufgebaut sein. Auch die Kombination mit strahlenhärtbaren Bindemitteln, wie sie beispielsweise in der EP-A-0 753 531 beschrieben sind, ist möglich. Ferner können auch andere anionische oder nichtionische Dispersionen, wie Polyvinylacetat-, Polyethylen-, Polystyrol-, Polybutadien-, Polyvinylchlorid-, Polyacrylat- oder Copolymerisat-Dispersionen eingesetzt werden.

Die Herstellung der mikroporösen Polyurethan-Schäume erfolgt, indem die Komponenten (I), (II) und gegebenenfalls (III) miteinander vermischt und gleichzeitig aufgeschäumt werden. Diese Homogenisierung kann beispielsweise mittels dynamischer Durchmischung oder im Statikmischer erfolgen, bevorzugt ist die Durchmischung mittels Statikmischer.

Das Aufschäumen im erfindungsgemäßen Verfahren geschieht beispielsweise durch mechanisches Rühren der Zusammensetzung bei hohen Drehzahlen, d.h. unter Eintrag hoher Scherkräfte, oder bevorzugt durch Entspannung eines Treibgases.

Als Treibgas können prinzipiell alle dem Fachmann an sich bekannten Treibmittel wie beispielsweise Kohlenwasserstoffe (beispielsweise Propan, Propen, n-Butan, iso-Butan, Buten, Pentan), Dimethylether, Dimethoxymethan, Kohlendioxid, Lachgas, Stickstoff, Sauerstoff, Edelgase, Luft und weniger bevorzugt FKW (z.B. R134a) und FCKW (z.B. Trichlorfluormethan, Dichlordifluormethan) sowie Mischungen dieser Gase verwendet werden. Bevorzugt werden Kohlenwasserstoffe, Dimethylether oder Kohlendioxid, besonders bevorzugt Kohlenwasserstoffe und ganz besonders bevorzugt Mischungen aus C3- und C4-Kohlenwasserstoffen eingesetzt.

Das Treibgas (-gemisch) kann sowohl einer der beiden Komponenten (I) oder (II) als auch beiden Komponenten zugesetzt werden. Der Zusatz von Treibgas kann eine Viskositätsveränderung der Komponente (I) bzw. (II) zur Folge haben. Denkbar ist auch der Zusatz unterschiedlicher Treibgase zu den beiden Dispersionen (I) und (II). Abhängig vom Treibgas resultiert ein ein- oder zweiphasiges Wasser-Gas-Gemisch, wobei das vollständige Lösen des Treibmittels in der Komponente (I) bzw. (II) bevorzugt ist.

Bezogen auf die wässrige Gesamtmasse der zu begasenden Dispersion (I) bzw. (II), jeweils ggf. Komponente (III) enthaltend, werden bevorzugt ≤30 Gewichtsteile bis ≥1 Gewichtsteile, besonders bevorzugt ≤20 Gewichtsteile bis ≥1 Gewichtsteile, ganz besonders bevorzugt ≤10 Gewichtsteile bis ≥3 Gewichtsteile Treibmittel zugesetzt.

Bezogen auf die resultierende wässrige Gesamtmasse der beiden miteinander zu mischenden Dispersionen (I) und (II), jeweils ggf. Komponente (III) enthaltend, werden bevorzugt ≥25 Gewichtsteile bis ≤90 Gewichtsteile, besonders bevorzugt ≥35 Gewichtsteile bis ≤80 Gewichtsteile, ganz besonders bevorzugt ≥45 Gewichtsteile bis ≤75 Gewichtsteile an Dispersion (I) und bevorzugt ≤75 Gewichtsteile bis ≥10 Gewichtsteile, besonders bevorzugt ≤65 Gewichtsteile bis ≥20 Gewichtsteile, ganz besonders bevorzugt ≤55 Gewichtsteile bis ≥25 Gewichtsteile an Dispersion (II) verwendet.

Bezogen auf das resultierende Gesamtvolumen der beiden miteinander zu mischenden Dispersionen (I) und (II), jeweils ggf. Komponente (III) enthaltend, werden bevorzugt ≥25 Volumenteile bis ≤90 Volumenteile, besonders bevorzugt ≥35 Volumenteile bis ≤80 Volumenteile, ganz besonders bevorzugt ≥45 Volumenteile bis ≤75 Volumenteile an Dispersion (I) und bevorzugt ≤75 Volumenteile bis ≥ 10 Volumenteile, besonders bevorzugt ≤65 Volumenteile bis ≥20 Volumenteile, ganz besonders bevorzugt ≤55 Volumenteile bis ≥25 Volumenteile an Dispersion (II) verwendet. Bevorzugt kommt es nach < 5 Minuten, besonders bevorzugt nach < 1 Minute und ganz besonders bevorzugt nach < 30 Sekunden zur Verfestigung der Schaumpaste. Bevorzugt kommt es erst nach 1 Sekunde, besonders bevorzugt erst nach 3 Sekunden und ganz besonders bevorzugt erst nach 5 Sekunden zur Verfestigung, um zunächst ein gleichmäßiges Verteilen oder Verlaufen der Schaumpaste zu ermöglichen.

Eine befriedigende Trocknungsgeschwindigkeit der Schäume wird in einer bevorzugten Ausführungsform bereits bei 20°C beobachtet, so dass eine Trocknung auf verletztem oder unverletztem menschlichen oder tierischen Gewebe problemlos möglich ist. Für eine schnellere Trocknung der Schäume können jedoch auch Temperaturen oberhalb von 30°C benutzt werden. Bei der Trocknung sollten jedoch Temperaturen von 200°C, bevorzugt 160°C, besonders bevorzugt 140°C nicht überschritten werden, da es anderenfalls u.a. zu unerwünschter Vergilbung der Schäume kommen kann. Möglich ist auch eine zwei- oder mehrstufige Trocknung. Weiterhin ist auch eine Trocknung unter dem Einfluss von Infrarotstrahlung oder Mikrowellenstrahlung möglich. Bei Trocknung auf menschlichem oder tierischem Gewebe ist es bevorzugt, auf Erwärmung durch externe Wärmezuführung zu verzichten.

Die Polyurethan-Schäume haben vor ihrer Trocknung typischerweise Schaumdichten von 50 bis 800 g/Liter, bevorzugt 100 bis 700 g/Liter, besonders bevorzugt 200 bis 600 g/Liter (Masse aller Einsatzstoffe [in g] bezogen auf das Schaumvolumen von einem Liter). Die Dichte der getrockneten Schäume liegt typischerweise bei 50 bis 800 g/Liter, bevorzugt bei 100 bis 600 g/Liter und liegt ganz besonders bevorzugt bei 200 bis 500 g/Liter.

Die Polyurethan-Schäume lassen sich in nahezu beliebiger Dicke herstellen. Typischerweise werden getrocknete Schäume mit einer Dicke von 0,1 mm bis 100 mm, bevorzugt 1 mm bis 30 mm, besonders bevorzugt 5 mm bis 20 mm, ganz besonders bevorzugt 5 bis 10 mm hergestellt. Auch ein Auftragen mehrerer Schichten der Polyurethan-Schäume hintereinander ist möglich, ggf. unter Aufbringen von Zwischenschichten, die nicht den erfindungsgemäßen Polyurethan-Schäumen entsprechen.

Unabhängig von der Trocknung verfestigen sich die erfindungsgemäßen Schäume innerhalb weniger Sekunden nach Applikation und es wird eine mikroporöse, zumindest teilweise offenporige Struktur mit miteinander kommunizierenden Zellen erhalten. Die Polyurethan-Schäume weisen bereits im noch feuchten Zustand eine gute mechanische Festigkeit und Elastizität auf. Typischerweise sind die Werte für die maximale Dehnung größer als 50 %, bevorzugt größer als 100 % (Bestimmung nach DIN 53504).

Die resultierenden, noch feuchten Schäume besitzen ein pH-Wert von ≥4 bis ≤9, bevorzugt von ≥ 5 bis ≤7, besonders bevorzugt von ≥5 bis ≤6.

Obgleich diese Schäume dispersionsbedingt bereits Wasser beinhalten, so sind sie dennoch in der Lage, zusätzliches Flüssigkeitsvolumen zu absorbieren. Es kommt zu keiner wesentlichen Quellung der hydrophilen Schäume.

Die erfindungsgemäßen Polyurethan-Schäume haben typischerweise während der Trocknung einen Volumenschrumpf von weniger als 30%, bevorzugt von weniger als 20%, besonders bevorzugt weniger als 10% bezogen auf das Schaumvolumen unmittelbar nach dem Schäumungsvorgang.

Die Durchlässigkeit gegenüber Wasserdampf der noch feuchten Schäume beträgt typischerweise 1000 bis 8000 g/24 h*m², bevorzugt 2000 bis 8000 g/24 h*m², besonders bevorzugt 3000 bis 8000 g/24 h*m² (Bestimmung nach DIN EN 13726-2, Teil 3.2).

Die erfindungsgemäßen Polyurethan-Schäume weisen in getrocknetem Zustand bei Exposition gegenüber isotonischer Kochsalzlösung bei 23°C eine Quellung von weniger als 5 %, bevorzugt weniger als 2 % auf.

Die erfindungsgemäßen Schäume können auch auf Substrate aufgebracht werden. Geeignet hierzu sind beispielsweise textile Flächengebilde, Flächensubstrate aus Metall, Glas, Keramik, Beton, Naturstein, Leder, Naturfasern, und Kunststoffen wie PVC, Polyolefine, Polyurethan oder ähnliches. Unter textilen Flächengebilden im Sinne der vorliegenden Erfindung sind beispielsweise Gewebe, Gewirke, gebundene und ungebundene Vliese zu verstehen. Die textilen Flächengebilde können aus synthetischen, natürlichen Fasern und/oder deren Mischungen aufgebaut sein. Grundsätzlich sind Textilien aus beliebigen Fasern für das erfindungsgemäße Verfahren geeignet.

Als Substrate geeignet sind aber auch insbesondere Papiere oder Folien, die ein einfaches Ablösen der Wundauflage vor ihrem Einsatz zur Abdeckung einer verletzten Stelle ermöglichen. Ebenso kann als Substrat menschliches oder tierisches Gewebe wie Haut dienen, so dass ein direktes Verschließen einer verletzten Stelle durch eine in-situ hergestellte Wundauflage möglich ist.

Die Polyurethan-Schäume können überdies mit weiteren Materialien verklebt, laminiert oder beschichtet werden, beispielsweise auf Basis von Hydrogelen, (semi-)permeablen Folien, Beschichtungen oder anderen Schäumen.

Es ist auch möglich, Wunden oder unverletzte Körperpartien zunächst mit einer Folie oder einem Papier auszulegen bzw. zu bedecken und dann den erfindungsgemäßen Schaum ohne Wundkontakt auszuhärten. Damit ließe sich ein direkter Wundkontakt bei der Aushärtung vermeiden. Ebenfalls ist es möglich, nach dem Aushärten die Trennfolie bzw. das Papier von der Wunde oder unverletzten Körperpartie zu entfernen, so dass nach dem Härten ein direkter Wundkontakt mit dem Schaum entsteht.

Es ist auch möglich, den Schaum nach der Durchmischung aber vor dem vollständigen Härten in eine Form zu expandieren und nach dem Härten die formgebenden Teile zu entfernen. Auf diese Weise können dreidimensional geformte Schäume gezielt hergestellt werden, beispielsweise, um sie einer Wunde oder auch einer ggf. unverletzten Körperpartie wie zum Beispiel der Ferse anzupassen.

### Beispiele:

Sofern nicht abweichend gekennzeichnet, beziehen sich alle Prozentangaben auf das Gewicht.

Die Bestimmung der Festkörpergehalte erfolgte nach DIN-EN ISO 3251.

NCO-Gehalte wurden, wenn nicht ausdrücklich anders erwähnt, volumetrisch gemäß DIN-EN ISO 11909 bestimmt.

Die angegebenen Viskositäten wurden mittels Rotationsviskosimetrie nach DIN 53019 bei 23°C mit einem Rotationsviskosimeter der Firma Anton Paar Germany GmbH, Ostfildern, DE bestimmt.

### Ladungsbestimmung

Eine Portion der Probe wird auf 0,0001g genau eingewogen (Masse typischerweise zwischen 0,2g und 1g, je nach Ladungsmenge) mit einer 5 Gew.%igen wässrigen Tensidlösung (Brij-96 V, Fluka, Buchs, Schweiz Produkt-Nr. 16011) und zweifach entionisiertem Wasser versetzt und nach Zusatz einer definierten Menge Salzsäure (0,1n, damit der Ansatz einen Start-pH-Wert von ca. pH 3 aufweist; KMF Laborchemie GmbH, Lohmar, Art.Nr.: KMF.01-044.1000) mit wässriger Natronlauge-Maßlösung (0,05n; Bernd Kraft GmbH, Duisburg, Art.Nr.: 01056.3000) titriert. Zusätzlich wird zur Differenzierung der Oberflächenladung und der Serumsladung ein Teil (ca. 30g) der Dispersion mit Ionenaustauscher Lewatit VP-OC 1293 (Einsatz der 10-fachen Austauschkapazität bezogen auf die bestimmte Gesamtladung, Rührzeit 2,0 h, Lanxess AG, Leverkusen, gemischter Anionen/Kationen-Austauscher) behandelt und die erhaltene Dispersion nach Filtration (E-D-Schnellsieb, Baumwollgewebe 240µm Fa. Erich Drehkopf GmbH, Ammersbek) titriert. Bei der Titration der Probe nach Ionenaustauscherbehandlung wird die Oberflächenladung bestimmt. Durch Differenzbildung mit der Gesamtladung kann die Serumladung ermittelt werden.

Die Bestimmung der Oberflächenladung aus den Equivalenzpunkten ergibt, innerhalb der Messgenauigkeit, einen vergleichbaren Wert zu der Ermittlung basischer Gruppen aus dem Minderverbrauch an Natronlauge, bezogen auf die zugesetzte Menge an Salzsäure.

### Verwendete Substanzen und Abkürzungen:

| | |
|---|---|
| Diaminosulfonat: | NH₂-CH₂CH₂-NH-CH₂CH₂-SO₃Na (45 %ig in Wasser) |
| Desmophen^{®} C2200: | Polycarbonatpolyol, OH-Zahl 56 mg KOH/g, zahlenmittleres Molekulargewicht 2000 g/mol (BayerMaterialScience AG, Leverkusen, DE) |
| PolyTHF^{®} 2000: | Polytetramethylenglykolpolyol, OH-Zahl 56 mg KOH/g, zahlenmittleres Molekulargewicht 2000 g/mol (BASF AG, Ludwigshafen, DE) |
| PolyTHF^{®} 1000: | Polytetramethylenglykolpolyol, OH-Zahl 112 mg KOH/g, zahlenmittleres Molekulargewicht 1000 g/mol (BASF AG, Ludwigshafen, DE) |
| Polyether LB 25: | monofunktioneller Polyether auf Ethylenoxid-/Propylenoxidbasis, zahlenmittleres Molekulargewicht 2250 g/mol, OH-Zahl 25 mg KOH/g (Bayer Material Science AG, Leverkusen, DE) |
| Pluronic^{®} PE3500: | Ethylenoxid-Propylenoxid-Blockcopolymer, Ethylenoxidanteil ca. 50%, zahlenmittleres Molekulargewicht ca. 1900 g/mol (BASF AG, Ludwigshafen, DE) |
| Pluronic^{®} PE6800: | Ethylenoxid-Propylenoxid-Blockcopolymer, Ethylenoxidanteil ca. 80%, zahlenmittleres Molekulargewicht ca. 8000 g/mol (BASF AG, Ludwigshafen, DE) |

### Beispiel 1: Polyurethan-Dispersion (I)

450 g PolyTHF^{®} 1000 und 2100 g PolyTHF^{®} 2000 wurden auf 70°C aufgeheizt. Anschließend wurde bei 70°C innerhalb von 5 min ein Gemisch aus 225,8 g Hexamethylendiisocyanat und 298,4 g Isophorondiisocyanat zugegeben und solange bei 100-115°C gerührt bis der theoretische NCO-Wert unterschritten war. Das fertige Prepolymer wurde mit 5460 g Aceton bei 50°C gelöst und anschließend eine Lösung aus 29,5 g Ethylendiamin, 143,2 g Diaminosulfonat und 610 g Wasser innerhalb von 10 min zudosiert. Die Nachrührzeit betrug 15 min. Danach wurde innerhalb von 10 min durch Zugabe von 1880 g Wasser dispergiert. Es folgte die Entfernung des Lösemittels durch Destillation im Vakuum (dabei wurde zusätzlich abdestilliertes Wasser ersetzt) und es wurde eine lagerstabile Dispersion mit nachfolgenden Eigenschaften erhalten:

| | |
|---|---|
| Feststoffgehalt: | 56 % |
| Partikelgröße (LKS): | 276 nm |
| pH (23°C): | 7,0 |
| Viskosität: | 1000 mPas |

### Beispiel 2: Polyurethan-Dispersion (I)

1165,5 g PolyTHF^{®} 2000, 250,3 g PolyTHF^{®} 1000 und 14,3 g Polyether LB 25 wurden in einer Standard-Rührapparatur auf 70 °C aufgeheizt. Anschließend wurde bei 70 °C innerhalb von 5 min ein Gemisch aus 158,0 g Hexamethylendiisocyanat und 208,8 g Isophorondiisocyanat zugegeben und bei 120°C gerührt, bis der theoretische NCO-Wert leicht unterschritten war. Das fertige Prepolymer wurde mit 4620 g Aceton gelöst und dabei auf 50 °C abgekühlt und anschließend wurde eine Lösung aus 16,8 g Ethylendiamin, 68,7 g Isophorondiamin, 63,5 g Diaminosulfonat und 730 g Wasser innerhalb von 10 min zudosiert. Die Nachrührzeit betrug 10 min. Dann wurde durch Zugabe von 383 g Wasser dispergiert. Es folgte die Entfernung des Lösemittels durch Destillation im Vakuum und es wurde eine lagerstabile Dispersion mit nachfolgenden Eigenschaften erhalten:

| | |
|---|---|
| Feststoffgehalt: | 58,1 % |
| Partikelgröße (LKS): | 446 nm |
| pH (23°C): | 7,2 |
| Viskosität (23°C): | 331 mPas |

### Beispiel 3: Polyurethan-Dispersion (I)

467,5 g eines OH-funktionellen Polyesters aus Adipinsäure, Hexandiol und Neopentylglykol mit einem mittleren Molekulargewicht von 1700 g/mol wurden auf 65°C aufgeheizt. Anschließend wurden bei 70°C innerhalb von 5 min eine Mischung von 54,7 g Isophorondiisocyanat und 64,6 g Methylen-bis-(4-isocyanatocyclohexan) zugegeben und solange bei 100-115°C gerührt bis der theoretische NCO-Wert unterschritten war. Das fertige Prepolymer wurde mit 1040 g Aceton bei 50°C gelöst und anschließend werden 7,9 g Methylen-bis-(4-aminocyclohexan) und nach weiteren 5 Minuten eine Lösung aus 1,0 g Ethylendiamin, 41,8 g Diaminosulfonat und 180 g Wasser innerhalb von 3 min zudosiert. Die Nachrührzeit betrug 5 min. Danach wurde innerhalb von 10 min durch Zugabe von 241 g Wasser dispergiert. Es folgte die Entfernung des Lösemittels durch Destillation im Vakuum unter Zusatz weiterer 370 g Wasser. Es wurde eine lagerstabile Dispersion mit nachfolgenden Eigenschaften erhalten:

| | |
|---|---|
| Feststoffgehalt: | 43 % |
| Partikelgröße (LKS): | 113 nm |
| pH-Wert: | 7,2 |
| Viskosität: | 410 mPas |

### Beispiel 4: Polyurethan-Dispersion (II)

882,3 g Desmophen C 2200 und 189,1 g PolyTHF^{®} 1000 wurden in einer Standard-Rührapparatur auf 70 °C aufgeheizt. Anschließend wurde bei 70 °C innerhalb von 5 min ein Gemisch aus 143 g Hexamethylendiisocyanat und 189 g Isophorondiisocyanat zugegeben und bei 120°C gerührt, bis der theoretische NCO-Wert leicht unterschritten war. Das Prepolymer wurde auf 80°C abgekühlt und dann mit 95,4 g N-Methyl-diethanolamin versetzt. Es wurde weitere 30 Minuten bei 80°C gerührt und dann mit 1500 g Aceton gelöst und dabei auf 50 °C abgekühlt. Anschließend wurde eine Lösung aus 17,1 g Isophorondiamin in 31 g Aceton und nach weiteren 10 Minuten 0,5 g Ethylendiamin in 2,3 g Wasser innerhalb von 10 min zudosiert. Die Nachrührzeit betrug 10 min. Dann wurde durch Zugabe von 86,9 g 85%iger wässriger Phosphorsäure neutralisiert und direkt im Anschluss mit 3450 g Wasser dispergiert. Es folgte die Entfernung des Lösemittels durch Destillation im Vakuum und eine lagerstabile Dispersion erhalten.

Die erhaltene Polyurethan-Dispersion hatte nachfolgende Eigenschaften:

| | |
|---|---|
| Feststoffgehalt: | 31,4% |
| Partikelgröße (LKS): | 87 nm |
| pH (23°C): | 4,4 |
| Viskosität (23°C): | 130 mPas |

### Beispiel 5: Polyurethan-Dispersion (II)

185,2 g Desmophen C 2200 und 39,7 g PolyTHF^{®} 1000 wurden in einer Standard-Rührapparatur auf 70 °C aufgeheizt. Anschließend wurde bei 70 °C innerhalb von 5 min ein Gemisch aus 30,0 g Hexamethylendiisocyanat und 39,6 g Isophorondiisocyanat zugegeben und bei 120°C gerührt, bis der theoretische NCO-Wert leicht unterschritten war. Das Prepolymer wurde auf 80°C abgekühlt und dann mit 20,0 g N-Methyl-diethanolamin versetzt. Es wurde weitere 30 Minuten bei 80°C gerührt und dann mit 315 g Aceton gelöst und dabei auf 50 °C abgekühlt. Anschließend wurde eine Lösung aus 3,6 g Isophorondiamin in 6,4 g Aceton und nach weiteren 10 Minuten 0,1 g Ethylendiamin in 0,5 g Wasser innerhalb von 10 min zudosiert. Die Nachrührzeit betrug 10 min. Dann wurde durch Zugabe von 143,4 g 1-normaler wässriger Salzsäure neutralisiert und direkt im Anschluss mit 600 g Wasser dispergiert. Es folgte die Entfernung des Lösemittels durch Destillation im Vakuum und eine lagerstabile Dispersion mit nachfolgenden Eigenschaften wurde erhalten:

| | |
|---|---|
| Feststoffgehalt: | 29,6 % |
| Partikelgröße (LKS): | 66 nm |
| pH (23°C): | 5,8 |
| Viskosität (23°C): | 30 mPas |

### Beispiel 6: Polyurethan-Dispersion (II)

185,2 g PolyTHF^{®} 2000 und 39,7 g PolyTHF^{®} 1000 wurden in einer Standard-Rührapparatur auf 70 °C aufgeheizt. Anschließend wurde bei 70 °C innerhalb von 5 min ein Gemisch aus 30,0 g Hexamethylendiisocyanat und 39,6 g Isophorondiisocyanat zugegeben und bei 120°C gerührt, bis der theoretische NCO-Wert leicht unterschritten war. Das Prepolymer wurde auf 80°C abgekühlt und dann mit 20,0 g N-Methyl-diethanolamin versetzt. Es wurde weitere 30 Minuten bei 80°C gerührt und dann mit 315 g Aceton gelöst und dabei auf 50 °C abgekühlt. Anschließend wurde eine Lösung aus 3,6 g Isophorondiamin in 6,4 g Aceton und nach weiteren 10 Minuten 0,1 g Ethylendiamin in 0,5 g Wasser innerhalb von 10 min zudosiert. Die Nachrührzeit betrug 10 min. Dann wurde durch Zugabe von 143,4 g 1-normaler wässriger Salzsäure neutralisiert und direkt im Anschluss mit 600 g Wasser dispergiert. Es folgte die Entfernung des Lösemittels durch Destillation im Vakuum und eine Dispersion mit nachfolgenden Eigenschaften wurde erhalten:

| | |
|---|---|
| Feststoffgehalt: | 31,0 % |
| Partikelgröße (LKS): | 180 nm |
| pH (23°C): | 6,1 |
| Viskosität (23°C): | 25 mPas |

### Beispiel 7: Koagulation zweier Dispersionen

400 g einer Dispersion gemäß Beispiel 1 wurden mit 6 g Pluronic^{®} PE6800 und 118 g Wasser versetzt. Anschließend wurde eine der beiden Kammern einer handelsüblichen Sprühdose mit Zwei-Kammer-Aerosoltechnologie mit 30 g der anionischen Dispersionsmischung und die andere Kammer mit 30 g einer kationischen Dispersion gemäß Beispiel 4 befüllt. Die Dose wurde mit ca. 3 g eines Propan/Butan-Gemisches als Treibmittel vorbegast. Zusätzlich wurde die die anionische Dispersionsmischung enthaltende Kammer mit etwa 1,5 g des gleichen Treibgases befüllt und die Dose anschließend verschlossen. Nach eintägiger Lagerung bei Raumtemperatur erfolgte das Versprühen der beiden mittels Statikmixer vermischten Komponenten. Als Mischungsverhältnis der beiden Komponenten zueinander wurde 2,1 g anionische PUD-Mischung zu 1,0 g kationische PUD-Mischung ermittelt.

Innerhalb weniger Sekunden nach dem Versprühen verfestigte sich der resultierende Schaum, ohne dass eine Wärmetönung (in Form einer Temperaturerhöhung) beobachtet wurde. Der erhaltene, reinweiße Schaum besaß bereits im noch feuchten Zustand eine Absorption von 130 Gew.% (Bestimmung nach DIN EN 13726-1, Teil 3.2) sowie eine Wasserdampfdurchlässigkeit von 4000 g/24 h*m² bei einer Schaumdicke von etwa 3-4 mm (Bestimmung nach DIN EN 13726-2, Teil 3.2). Auch nach vollständiger Trocknung und erneuter Flüssigkeitsabsorption zeigte er keine merkliche Quellung.

### Beispiel 8: Koagulation zweier Dispersionen

Eine der beiden Kammern einer handelsüblichen Sprühdose mit Zwei-Kammer-Aerosoltechnologie wurde mit 30 g einer anionischen Dispersion gemäß Beispiel 2 und die andere Kammer mit 30 g einer kationischen Dispersionsmischung gemäß Beispiel 5 befüllt. Die Dose wurde mit ca. 3 g eines Propan/Butan-Gemisches als Treibmittel vorbegast. Zusätzliche wurde die die anionische Dispersionsmischung enthaltende Kammer mit etwa 1,5 g des gleichen Treibgases befüllt und die Dose anschließend verschlossen. Nach eintägiger Lagerung bei Raumtemperatur erfolgte das Versprühen der beiden mittels Statikmixer vermischten Komponenten. Als Mischungsverhältnis der beiden Komponenten zueinander wurde 1,0 g anionische PUD-Mischung zu 1,0 g kationische PUD-Mischung ermittelt.

Innerhalb weniger Sekunden nach dem Versprühen verfestigte sich der resultierende Schaum, ohne dass eine Wärmetönung beobachtet wurde. Der erhaltene, reinweiße Schaum besaß bereits im noch feuchten Zustand eine merkliche Absorption für isotonische Kochsalzlösung sowie eine gute Wasserdampfdurchlässigkeit. Auch nach vollständiger Trocknung und erneuter Flüssigkeitsabsorption zeigte er keine merkliche Quellung.

### Beispiel 9: Koagulation zweier Dispersionen

400 g einer Dispersion gemäß Beispiel 6 wurden mit 1,2 g Pluronic^{®} PE3500 versetzt. Anschließend wurde eine der beiden Kammern einer handelsüblichen Sprühdose mit Zwei-Kammer-Aerosoltechnologie mit 30 g dieser Dispersionsmischung und die andere Kammer mit 30 g einer Dispersion gemäß Beispiel 3 befüllt. Die Dose wurde mit ca. 3 g eines Propan/Butan-Gemisches als Treibmittel vorbegast. Zusätzliche wurde die die anionische Dispersionsmischung enthaltende Kammer mit etwa 1,5 g des gleichen Treibgases befüllt und die Dose anschließend verschlossen. Nach eintägiger Lagerung bei Raumtemperatur erfolgte das Versprühen der beiden mittels Statikmixer vermischten Komponenten. Als Mischungsverhältnis der beiden Komponenten zueinander wurde 1,2:1 (anionische PUD-Mischung : kationische PUD-Mischung) ermittelt.

Innerhalb weniger Sekunden nach dem Versprühen verfestigte sich der resultierende Schaum, ohne dass eine Wärmetönung beobachtet wurde. Der erhaltene, reinweiße Schaum besaß bereits im noch feuchten Zustand eine merkliche Absorption für Salzlösungen sowie eine Wasserdampfdurchlässigkeit von 4600 g/24 h*m² bei einer Schaumdicke von etwa 3-4 mm (Bestimmung nach DIN EN 13726-2, Teil 3.2). Auch nach vollständiger Trocknung und erneuter Flüssigkeitsabsorption zeigte er keine merkliche Quellung.

## Patentansprüche

1. Verwendung von Polyurethan-Schäumen erhältlich nach einem Verfahren, bei dem eine Zusammensetzung erhältlich durch Vermischen von wenigstens einer wässrigen, anionisch hydrophilierten Polyurethan-Dispersion (I) und wenigstens einer kationisch hydrophilierten PUR-Dispersion (II) bereitgestellt und während oder nach der vollständigen Vermischung der Einzelkomponenten aufgeschäumt und ausgehärtet wird, als Wundauflage.

2. Verwendung gemäß Anspruch 1, **dadurch gekennzeichnet, dass** die wässrigen, anionisch hydrophilierten Polyurethan-Dispersionen (I) erhältlich sind, indem
A) isocyanatfunktionelle Prepolymere aus
A1) organischen Polyisocyanaten
A2) polymeren Polyolen mit zahlenmittleren Molekulargewichten von 400 bis 8000 g/mol, vorzugsweise 400 bis 6000 g/mol und besonders bevorzugt von 600 bis 3000 g/mol, und OH-Funktionalitäten von 1,5 bis 6, bevorzugt 1,8 bis 3, besonders bevorzugt von 1,9 bis 2,1, und
A3) gegebenenfalls hydroxyfunktionellen Verbindungen mit Molekulargewichten von 62 bis 399 g/mol und
A4) gegebenenfalls isocyanatreaktiven, anionischen oder potentiell anionischen und/oder gegebenenfalls nichtionischen Hydrophitierungsmitteln,
hergestellt werden,
B) deren freie NCO-Gruppen dann ganz oder teilweise
B1) gegebenenfalls mit aminofunktionellen Verbindungen mit Molekulargewichten von 32 bis 400 g/mol und
B2) gegebenenfalls mit isocyanatreaktiven, bevorzugt aminofunktionellen, anionischen oder potentiell anionischen Hydrophilierungsmitteln
unter Kettenverlängerung umgesetzt werden und die Prepolymere vor während oder nach Schritt B) in Wasser dispergiert werden, wobei gegebenenfalls enthaltene potentiell ionische Gruppen durch teilweise oder vollständige Umsetzung mit einem Neutralisationsmittel in die ionische Form überführt werden.

3. Verwendung gemäß Anspruch 1 oder 2, **dadurch gekennzeichnet, dass** die wässrigen, kationisch oder potentiell kationisch hydrophilierten Polyurethan-Dispersionen (II) erhältlich sind, indem
C) isocyanafunktionelle Prepolymere aus
C1) organischen Polyisocyanaten
C2) polymeren Polyolen
C3) gegebenenfalls hydroxyfunktionellen Verbindungen und
C4) gegebenenfalls isocyanatreaktiven Verbindungen, die kationische Gruppen oder in kationische Gruppen überführbare Einheiten enthalten und/oder gegebenenfalls nichtionisch hydrophilierenden Verbindungen enthalten
hergestellt werden,
D) deren freie NCO-Gruppen dann ganz oder teilweise
D1) gegebenenfalls mit den in B1) beschriebenen aminofunktionellen Verbindungen und
D2) gegebenenfalls mit isocyanatreaktiven, bevorzugt aminofunktionellen, kationischen oder potentiell kationischen hydrophilierungsmitteln
unter Kettenverlängerung umgesetzt werden und die Prepolymere vor während oder nach Schritt D) in Wasser dispergiert werden, wobei gegebenenfalls enthaltene potentiell ionische Gruppen durch teilweise oder vollständige Umsetzung mit einem Neutralisutionsmittel in die ionische Form überfährt werden.

4. Verwendung gemäß einem der Ansprüche 1 bis 3, **dadurch gekennzeichnet, dass** die wässrigen Polyurethan-Dispersion (I) und (II) jeweils einen Gehalt an ionischen Gruppen von 2 bis 200 Milliäquivalenten pro 100 g Festharz der jeweiligen Dispersion aufweisen.

5. Verwendung gemäß einem der Ansprüche 1 bis 4, **dadurch gekennzeichnet, dass** als Komponente A2) und/oder C2) eine Mischung aus Polycarbonatpolyolen und Polytetramethylenglykolpolyolen eingesetzt wird, wobei in dieser Mischung der Anteil an Polycarbonatpolyolen 0 bis 80 Gew.-% und der Anteil an Polytetramethylenglykolpolyolert I00 bis 20 Gew.-% beträgt.

6. Verwendung gemäß einem der Ansprüche 1 bis 5, **dadurch gekennzeichnet, dass** in A1) und/oder C1) Polyisocyanate oder Polyisocyanatgemische mit ausschließlich aliphatisch und/oder cycloaliphatisch gebundenen Isocyanatgruppen und einer mittleren NCO-Funktionalität der Mischung von 2 bis 4 eingesetzt wird.

7. Verwendung gemäß einem der Ansprüche 1 bis 6, **dadurch gekennzeichnet, dass** diese ferner Hilfs - und Zusatzstoffe (III) mitumfassen.

8. Verwendung nach einem der Ansprüche 1 bis 7, **dadurch gekennzeichnet, dass** die Aushärtung sowohl eine Verfestigung des Schaumes als auch einen anschließenden Trocknungsschritt des Schaumes umfasst.

9. Verwendung nach einem der Ansprüche 1 bis 8, **dadurch gekennzeichnet, dass** diese eine Dichte in verfestigtem und getrocknetem Zustand von 50 bis 800 g/Liter aufweisen.

10. Verwendung nach einem der Ansprüche 1 bis 9, **dadurch gekennzeichnet, dass** diese eine Durchlässigkeit gegenüber Wasserdampf von 1000 bis 8000 g/24 h*m² (Bestimmung nach DIN EN 13726-2, Teil 3.2) aufweisen.

## Claims

1. Use of polyurethane foams obtainable by a process in which a composition obtainable by mixing at least one aqueous anionically hydrophilicized polyurethane dispersion (I) and at least one cationically hydrophilicized PU dispersion (II) is provided and, during or after the complete mixing of the individual components, frothed and cured, as a wound contact material.

2. Use according to Claim 1, **characterized in that** the aqueous anionically hydrophilicized polyurethane dispersions (I) are obtainable by
A) isocyanate-functional prepolymers being produced from
A1) organic polyisocyanates
A2) polymeric polyols having number average molecular weights in the range from 400 to 8000 g/mol, preferably in the range from 400 to 6000 g/mol and more preferably in the range from 600 to 3000 g/mol, and OH functionalities in the range from 1.5 to 6, preferably in the range from 1.8 to 3 and more preferably in the range from 1.9 to 2.1, and
A3) optionally hydroxyl-functional compounds having molecular weights in the range from 62 to 399 g/mol and
A4) optionally isocyanate-reactive, anionic or potentially anionic and/or optionally nonionic hydrophilicizing agents,
B) their free NCO groups then being wholly or partly reacted
B1) optionally with amino-functional compounds having molecular weights in the range from 32 to 400 g/mol and
B2) optionally with isocyanate-reactive, preferably amino-functional, anionic or potentially anionic hydrophilicizing agents
by chain extension, and the prepolymers being dispersed in water before, during or after step B), any potentially ionic groups present being converted into the ionic form by partial or complete reaction with a neutralizing agent.

3. Use according to Claim 1 or 2, **characterized in that** the aqueous cationically or potentially cationically hydrophilicized polyurethane dispersions (II) are obtainable by
C) isocyanate-functional prepolymers being produced from
C1) organic polyisocyanates
C2) polymeric polyols
C3) optionally hydroxyl-functional compounds and
C4) optionally isocyanate-reactive compounds that contain cationic groups or units convertible into cationic groups and/or optionally contain nonionically hydrophilicizing compounds,
D) their free NCO groups then being wholly or partly reacted
D1) optionally with the amino-functional compounds described in B1) and
D2) optionally with isocyanate-reactive, preferably amino-functional, cationic or potentially cationic hydrophilicizing agents
by chain extension, and the prepolymers being dispersed in water before, during or after step D), any potentially ionic groups present being converted into the ionic form by partial or complete reaction with a neutralizing agent.

4. Use according to any one of Claims 1 to 3, **characterized in that** the aqeous polyurethane dispersions (I) and (II) each have an ionic group content of 2 to 200 milliequivalents per 100 g of solid resin in the respective dispersion.

5. Use according to any one of Claims I to 4, **characterized in that** a mixture of polycarbonate polyols and polytetramethylene glycol polyols is utilized as component A2) and/or C2), the proportion of polycarbonate polyols in this mixture being in the range from 0% to 80% by weight and the proportion of polytetramethylene glycol polyols in this mixture being in the range from 100% to 20% by weight.

6. Use according to any one of Claims 1 to 5, **characterized in that** A1) and/or C1) utilize polyisocyanates or polyisocyanate mixtures having exclusively aliphatically and/or cycloaliphatically attached isocyanate groups and an average NCO functionality in the range from 2 to 4 for the mixture.

7. Use according to any one of Claims 1 to 6, **characterized in that** these further comprise auxiliary and adjunct materials (III).

8. Use according to any one of Claims 1 to 7, **characterized in that** the curing comprises not only a consolidation of the foam but also a subsequent drying step of the foam.

9. Use according to any one of Claims 1 to 8, **characterized in that** these have a density in the consolidated and dried state in the range from 50 to 800 g/litre.

10. Use according to any one of Claims 1 to 9, **characterized in that** these have a DIN EN 13726-2 Part 3.2 water vapour transmission rate in the range from 1000 to 8000 g/24 h*m².

## Revendications

1. Utilisation de mousses de polyuréthane pouvant être obtenues selon un procédé dans lequel on prépare une composition pouvant être obtenue par mélange d'au moins une dispersion aqueuse de polyuréthane (I) hydrophilisé par voie anionique et d'au moins une dispersion de polyuréthane (II) hydrophilisé par voie cationique et pendant ou après le mélange complet des composants individuels on la transforme en mousse et la fait durcir, en tant que pansement.

2. Utilisation selon la revendication 1, **caractérisée en ce que** les dispersions aqueuses de polyuréthane (I) hydrophilisé par voie anionique peuvent être obtenues par
A) préparation de prépolymères à fonction isocyanate à partir
A1) de polyisocyanates organiques
A2) de polyols polymères ayant des masses moléculaires moyennes en nombre de 400 à 8 000 g/mole, de préférence de 400 à 6 000 g/mole et de façon particulièrement préférée de 600 à 3 000 g/mole et des fonctionnalités OH de 1,5 à 6, de préférence de 1,8 à 3, de façon particulièrement préférée de 1,9 à 2,1, et
A3) éventuellement de composés à fonction hydroxy ayant des masses moléculaires de 62 à 399 g/mole et
A4) éventuellement d'agents d'hydrophilisation réactifs avec des isocyanates, anioniques ou potentiellement anioniques et/ou éventuellement non ioniques,
B) ensuite mise en réaction en partie ou en totalité de leurs groupes NCO libres
B1) éventuellement avec des composés à fonction amino, ayant des masses moléculaires de 32 à 400 g/mole et
B2) éventuellement avec des agents d'hydrophilisation de préférence à fonction amino, anioniques ou potentiellement anioniques, réactifs avec des isocyanates
avec prolongement de chaîne, et dispersion dans l'eau des prépolymères avant, pendant ou après l'étape B), des groupes potentiellement ioniques éventuellement contenus étant convertis en la forme ionique par mise en réaction partielle ou totale avec un agent de neutralisation.

3. Utilisation selon la revendication 1 ou 2, **caractérisée en ce que** les dispersions aqueuses de polyuréthane (II) hydrophilisé par voie cationique ou potentiellement cationique peuvent être obtenues par
C) préparation de prépolymères à fonction isocyanate à partir
C1) de polyisocyanates organiques
C2) de polyols polymères
C3) éventuellement de composés à fonction hydroxy et
C4) éventuellement de composés réactifs avec des isocyanates, qui contiennent des groupes cationiques ou des unités pouvant être converties en groupes cationiques et/ou éventuellement des composés hydrophilisant par voie non ionique,
D) ensuite mise en réaction en partie ou en totalité de leurs groupes NCO libres
D1) éventuellement avec les composés à fonction amino décrits en B1) et
D2) éventuellement avec des agents d'hydrophilisation de préférence à fonction amino, cationiques ou potentiellement cationiques, réactifs avec des isocyanates
avec prolongement de chaîne, et dispersion dans l'eau des prépolymères avant, pendant ou après l'étape D), des groupes potentiellement ioniques éventuellement contenus étant convertis en la forme ionique par mise en réaction partielle ou totale avec un agent de neutralisation.

4. Utilisation selon l'une quelconque des revendications 1 à 3, **caractérisée en ce que** les dispersions aqueuses de polyuréthane (I) et (II) présentent chacune une teneur en groupes ioniques de 2 à 200 milliéquivalents pour 100 g de résine solide de la dispersion respective.

5. Utilisation selon l'une quelconque des revendications 1 à 4, **caractérisée en ce qu'**on utilise comme composant A2) et/ou composant C2) un mélange de polycarbonatepolyols et polytétraméthylèneglycolpolyols, dans ce mélange la proportion des polycarbonatepolyols valant de 0 à 80 % en poids et la proportion des polytétraméthylèneglycolpolyols valant de 100 à 20 % en poids.

6. Utilisation selon l'une quelconque des revendications 1 à 5, **caractérisée en ce qu'**en A1) et/ou C1) on utilise des polyisocyanates ou mélanges de polyisocyanates comportant exclusivement des groupes isocyanate en liaison aliphatique et/ou cycloaliphatique et ayant une fonctionnalité NCO moyenne du mélange de 2 à 4.

7. Utilisation selon l'une quelconque des revendications 1 à 6, **caractérisée en ce que** ces compositions comprennent en même temps des adjuvants et additifs (III).

8. Utilisation selon l'une quelconque des revendications 1 à 7, **caractérisée en ce que** le durcissement comprend aussi bien une solidification de la mousse qu'une étape subséquente de séchage de la mousse.

9. Utilisation selon l'une quelconque des revendications 1 à 8, **caractérisée en ce que** ces mousses présentent une densité à l'état solidifié et séché de 50 à 800 g/litre.

10. Utilisation selon l'une quelconque des revendications 1 à 9, **caractérisée en ce que** ces mousses présentent une perméabilité à la vapeur d'eau de 1 000 à 8 000 g/24 h*m² (détermination selon DIN EN 13726-2, section 3.2).
